# EUROPEAN PATENT SPECIFICATION

(11) **EP 1 648 871 B1**
(45) Date of publication and mention of the grant of the patent: **17.10.2007**
(21) Application number: 04749171.7
(22) Date of filing: 14.07.2004
(51) Int. Cl.: C07D 221/24, A61K 31/445, A61P 29/00

(54) **PIPERIDINE OR 8-AZA-BICYCLO 3.2.1 OCT-3-YL DERIVATIVES USEFUL AS MODULATORS OF CHEMOKINE RECEPTOR ACTIVITY**
PIPERIDIN- ODER 8-AZABICYCLO[3.2.1]OCT-3-YLDERIVATE, DIE SICH ALS MODULATOREN DER CHEMOKINREZEPTORAKTIVITÄT EIGNEN
DERIVES DE PIPERIDINE OU 8-AZA-BICYCLO 3.2.1|OCT-3-YL QUE L'ON UTILISE COMME MODULATEURS DE L'ACTIVITE DU RECEPTEUR DES CHIMIOKINES

(30) Priority: 16.07.2003 SE 0302090
(43) Date of publication of application: 26.04.2006
(73) Proprietor: AstraZeneca AB, 151 85 Södertälje (SE)
(72) Inventor: BROWN, Dearg, AstraZeneca R & D Alderley, Macclesfield, Cheshire SK10 4TG (GB); CUMMING, John, AstraZeneca R & D Alderley, Macclesfield, Cheshire SK10 4TG (GB); TUCKER, Howard, AstraZeneca R & D Alderley, Macclesfield, Cheshire SK10 4TG (GB)
(86) International application number: PCT/SE2004/001135
(87) International publication number: WO 2005/007629

(56) References cited:
- WO-A1-03/042177
- WO-A1-03/042205
- WO-A1-03/080574

## Description

The present invention relates to heterocyclic derivatives having pharmaceutical activity, to processes for preparing such derivatives, to pharmaceutical compositions comprising such derivatives and to the use of such derivatives as active therapeutic agents.

Pharmaceutically active piperidine derivatives are disclosed in WO01/87839, EP-A1-1013276, WO00/08013, WO99/38514, WO99/04794, WO03/042205, WO03/042177 and WO04/056773.

Chemokines are chemotactic cytokines that are released by a wide variety of cells to attract macrophages, T cells, eosinophils, basophils and neutrophils to sites of inflammation and also play a rôle in the maturation of cells of the immune system. Chemokines play an important rôle in immune and inflammatory responses in various diseases and disorders, including asthma and allergic diseases, as well as autoimmune pathologies such as rheumatoid arthritis and atherosclerosis. These small secreted molecules are a growing superfamily of 8-14 kDa proteins characterised by a conserved four cysteine motif. The chemokine superfamily can be divided into two main groups exhibiting characteristic structural motifs, the Cys-X-Cys (C-X-C, or α) and Cys-Cys (C-C, or β) families. These are distinguished on the basis of a single amino acid insertion between the NH-proximal pair of cysteine residues and sequence similarity.

The C-X-C chemokines include several potent chemoattractants and activators of neutrophils such as interleukin-8 (IL-8) and neutrophil-activating peptide 2 (NAP-2).

The C-C chemokines include potent chemoattractants of monocytes and lymphocytes but not neutrophils such as human monocyte chemotactic proteins 1-3 (MCP-1, MCP-2 and MCP-3), RANTES (Regulated on Activation, Normal T Expressed and Secreted), eotaxin and the macrophage inflammatory proteins 1α and 1β (MIP-1α and MIP-1β).

Studies have demonstrated that the actions of the chemokines are mediated by subfamilies of G protein-coupled receptors, among which are the receptors designated CCR1, CCR2, CCR2A, CCR2B, CCR3, CCR4, CCR5, CCR6, CCR7, CCR8, CCR9, CCR10, CXCR1, CXCR2, CXCR3 and CXCR4. These receptors represent good targets for drug development since agents which modulate these receptors would be useful in the treatment of disorders and diseases such as those mentioned above.

The CCR5 receptor is expressed on T-lymphocytes, monocytes, macrophages, dendritic cells, microglia and other cell types. These detect and respond to several chemokines, principally "regulated on activation normal T-cell expressed and secreted" (RANTES), macrophage inflammatory proteins (MIP) MIP-1α and MIP-1β and monocyte chemoattractant protein-2 (MCP-2).

This results in the recruitment of cells of the immune system to sites of disease. In many diseases it is the cells expressing CCR5 which contribute, directly or indirectly, to tissue damage. Consequently, inhibiting the recruitment of these cells is beneficial in a wide range of diseases.

CCR5 is also a co-receptor for H1V-1 and other viruses, allowing these viruses to enter cells. Blocking the receptor with a CCR5 antagonist or inducing receptor internalisation with a CCR5 agonist protects cells from viral infection.

The present invention provides a compound of formula (I): 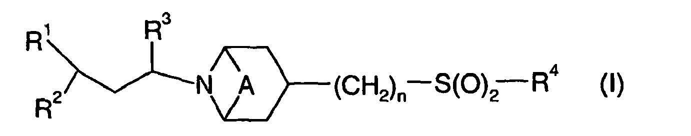 wherein:
A is absent or is (CH₂)₂;
R¹ is C(O)NR¹⁰R¹¹, C(O)₂R¹², NR¹³C(O)R¹⁴, NR¹⁵C(O)NR¹⁶R¹⁷, NR¹⁸C(O)₂R¹⁹, heterocyclyl (for example piperidine, piperazine, pyrrolidine or azetidine), aryl or heteroaryl;
R¹⁰, R¹³, R¹⁵, R¹⁶ and R¹⁸ are hydrogen or C₁₋₆ alkyl;
R¹¹, R¹², R¹⁴, R¹⁷ and R¹⁹ are C₁₋₈ alkyl (optionally substituted by halo, hydroxy, C₁₋₆ alkoxy, C₁₋₆ haloalkoxy, C₃₋₆ cycloalkyl (optionally substituted by halo), C₅₋₆ cycloalkenyl, S(C₁₋₄ alkyl), S(O)(C₁₋₄ alkyl), S(O)₂(C₁₋₄ alkyl), heteroaryl, aryl, heteroaryloxy or aryloxy), aryl, heteroaryl, C₃₋₇ cycloalkyl (optionally substituted by halo or C₁₋₄ alkyl), C₄₋₇ cycloalkyl fused to a phenyl ring, C₅₋₇ cycloalkenyl, or, heterocyclyl (itself optionally substituted by oxo, C(O)(C₁₋₆ alkyl), S(O)ₖ(C₁₋₆ alkyl), halo or C₁₋₄ alkyl); or R¹¹, R¹², R¹⁴ and R¹⁷ can also be hydrogen;
or R¹⁰ and R¹¹, and/or R¹⁶ and R¹⁷ may join to form a 4-, 5- or 6-membered ring which optionally includes a nitrogen, oxygen or sulphur atom, said ring being optionally substituted by C₁₋₆ alkyl, S(O)₁(C₁₋₆ alkyl) or C(O)(C₁₋₆ alkyl);
R² is phenyl, heteroaryl or C₃₋₇ cycloalkyl;
R³ is H or C₁₋₄ alkyl;
R⁴ is heterocyclyl;
n is 1, 2 or 3;
aryl, phenyl and heteroaryl moieties are independently optionally substituted by one or more of halo, cyano, nitro, hydroxy, OC(O)NR²⁰R²¹, NR²²R²³, NR²⁴C(O)R²⁵, NR²⁶C(O)NR²⁷R²⁸, S(O)₂NR²⁹R³⁰, NR³¹S(O)₂R³², C(O)NR³³R³⁴, CO₂R³⁶, NR³⁷CO₂R³⁸, S(O)_{q}R³⁹, OS(O)₂R⁴⁹, C₁₋₆ alkyl (optionally mono-substituted by S(O)₂R⁵⁰ or C(O)NR⁵¹R⁵²), C₂₋₆ alkenyl, C₂₋₆ alkynyl, C₃₋₁₀ cycloalkyl, C₁₋₆ haloalkyl, C₁₋₆ alkoxy(C₁₋₆)alkyl, C₁₋₆ alkoxy, C₁₋₆ haloalkoxy, phenyl, phenyl(C₁₋₄)alkyl, phenoxy, phenylthio, phenylS(O), phenylS(O)₂, phenyl(C₁₋₄)alkoxy, heteroaryl, heteroaryl(C₁₋₄)alkyl, heteroaryloxy or heteroaryl(C₁₋₄)alkoxy; wherein any of the immediately foregoing phenyl and heteroaryl moieties are optionally substituted with halo, hydroxy, nitro, S(C₁₋₄ alkyl), S(O)(C₁₋₄ alkyl), S(O)₂(C₁₋₄ alkyl), S(O)₂NH₂, S(O)₂NH(C₁₋₄ alkyl), S(O)₂N(C₁₋₄ alkyl)₂, cyano, C₁₋₄ alkyl, C₁₋₄ alkoxy, C(O)NH₂, C(O)NH(C₁₋₄ alkyl), C(O)N(C₁₋₄ alkyl)₂, CO₂H, CO₂(C₁₋₄ alkyl), NHC(O)(C₁₋₄ alkyl), NHS(O)₂(C₁₋₄ alkyl), CF₃ or OCF₃;
unless otherwise stated heterocyclyl is optionally substituted by C₁₋₆ alkyl [optionally substituted by phenyl {which itself optionally substituted by halo, C₁₋₄ alkyl, C₁₋₄ alkoxy, cyano, nitro, CF₃, OCF₃, (C₁₋₄ alkyl)C(O)NH, S(O)₂NH₂, C₁₋₄ alkylthio, S(O)(C₁₋₄ alkyl) or S(O)₂(C₁₋₄ alkyl)} or heteroaryl {which itself optionally substituted by halo, C₁₋₄ alkyl, C₁₋₄ alkoxy, cyano, nitro, CF₃, (C₁₋₄ alkyl)C(O)NH, S(O)₂NH₂, C₁₋₄ alkylthio, S(O)(C₁₋₄ alkyl) or S(O)₂(C₁₋₄ alkyl)}], phenyl {optionally substituted by halo, C₁₋₄ alkyl, C₁₋₄ alkoxy, cyano, nitro, CF₃, OCF₃, (C₁₋₄ alkyl)C(O)NH, S(O)₂NH₂, C₁₋₄ alkylthio, S(O)(C₁₋₄ alkyl) or S(O)₂(C₁₋₄ alkyl)}, heteroaryl {optionally substituted by halo, C₁₋₄ alkyl, C₁₋₄ alkoxy, cyano, nitro, CF₃, (C₁₋₄ alkyl)C(O)NH, S(O)₂NH₂, C₁₋₄ alkylthio, S(O)(C₁₋₄ alkyl) or S(O)₂(C₁₋₄ alkyl)}, S(O)₂NR⁴⁰R⁴¹, C(O)R⁴², C(O)₂(C₁₋₆ alkyl) (such as tert-butoxycarbonyl), C(O)₂(phenyl(C₁₋₂ alkyl)) (such as benzyloxycarbonyl), C(O)NHR⁴³, S(O)₂R⁴⁴, NHS(O)₂NHR⁴⁵, NHC(O)R⁴⁶, NHC(O)NHR⁴⁷ or NHS(O)₂R⁴⁸, provided none of these last four substituents is linked to a ring nitrogen;
k, l and q are, independently, 0, 1 or 2;
R²⁰, R²², R²⁴, R²⁶, R²⁷, R³¹, R³³, R³³, R³⁷, R⁴⁰ and R⁵¹ are, independently, hydrogen or C₁₋₆ alkyl;
R²¹, R²³, R²⁵, R²⁸, R³⁰, R³², R³⁴, R³⁶, R³⁸, R³⁹, R⁴¹, R⁴², R⁴³, R⁴⁴, R⁴⁵, R⁴⁶, R⁴⁷, R⁴⁸, R⁴⁹, R⁵⁰ and R⁵² are, independently, C₁₋₆ alkyl (optionally substituted by halo, hydroxy, C₁₋₆ alkoxy, C₁₋₆ haloalkoxy, C₃₋₆ cycloalkyl, C₅₋₆ cycloalkenyl, S(C₁₋₄ alkyl), S(O)(C₁₋₄ alkyl), S(O)₂(C₁₋₄ alkyl), heteroaryl, phenyl, heteroaryloxy or phenyloxy), C₃₋₇ cycloalkyl, phenyl or heteroaryl;
wherein any of the immediately foregoing phenyl and heteroaryl moieties are optionally substituted with halo, hydroxy, nitro, S(C₁₋₄ alkyl), S(O)(C₁₋₄ alkyl), S(O)₂(C₁₋₄ alkyl), S(O)₂NH₂, S(O)₂NH(C₁₋₄ alkyl), S(O)₂N(C₁₋₄ alkyl)₂, cyano, C₁₋₄ alkyl, C₁₋₄ alkoxy, C(O)NH₂, C(O)NH(C₁₋₄ alkyl), C(O)N(C₁₋₄ alkyl)₂, CO₂H, CO₂(C₁₋₄ alkyl), NHC(O)(C₁₋₄ alkyl), NHS(O)₂(C₁₋₄ alkyl), C(O)(C₁₋₄ alkyl), CF₃ or OCF₃;
R²¹, R²³, R²⁵, R²⁸, R³⁰, R³⁴, R³⁵, R³⁶, R⁴¹, R⁴², R⁴³, R⁴⁵, R⁴⁶, R⁴⁷ and R⁵² may additionally be hydrogen;
or a pharmaceutically acceptable salt thereof or a solvate thereof.

Certain compounds of the present invention can exist in different isomeric forms (such as enantiomers, diastereomers, geometric isomers or tautomers). The present invention covers all such isomers and mixtures thereof in all proportions.

Suitable salts include acid addition salts such as a hydrochloride, hydrobromide, phosphate, acetate, fumarate, maleate, tartrate, citrate, oxalate, methanesulphonate or p-toluenesulphonate.

The compounds of the invention may exist as solvates (such as hydrates) and the present invention covers all such solvates.

Alkyl groups and moieties are straight or branched chain and, for example, comprise one to six (such as one to four) carbon atoms. Alkyl is, for example, methyl, ethyl, n-propyl, iso-propyl, n-butyl, sec-butyl or tert-butyl. Methyl is sometimes abbreviated to Me hereinbelow.

Haloalkyl includes CF₃, and haloalkoxy includes OCF₃.

Fluoroalkyl includes, for example, one to six, such as one to three, fluorine atoms, and comprises, for example, a CF₃ group. Fluoroalkyl is, for example, CF₃ or CH₂CF₃.

Cycloalkyl is, for example, cyclopropyl, cyclopentyl or cyclohexyl (such as cyclohexyl). Cycloalkenyl includes cyclopentenyl.

Heterocyclyl is non-aromatic and is linked by a ring-carbon or ring-heteroatom (such as a ring-nitrogen), and is, for example, a 3-7-membered ring comprising at least one nitrogen, oxygen or sulphur atom. In one embodiment of the invention heterocyclyl has an oxygen atom; a sulphur atom; or, a nitrogen atom and, optionally, an oxygen atom or a sulphur atom. Heterocyclyl is, for example, piperidine, piperazine, pyrrolidine, azetidine, tetrahydrofuran, morpholine or thiomorpholine. In one aspect of the invention heterocyclyl is piperidinyl, homopiperazinyl, thiomorpholinyl, pyrrolidinyl, piperazinyl, 1,2,3,6-tetrahydropyiidinyl, morpholinyl, 2,5-dihydropyrrolyl, azetidinyl, 1,4-oxepanyl, 3-azabicyclo[3.2.1]octan-3-yl, 8-azaspiro[4.5]decanyl or 3-azabicyclo[3.1.0]hex-3-yl.

Aryl includes phenyl and naphthyl. In one aspect of the invention aryl is phenyl.

Heteroaryl is, for example, an aromatic 5 or 6 membered ring, optionally fused to one or more other rings, comprising at least one heteroatom selected from the group comprising nitrogen, oxygen and sulphur; or an N-oxide thereof, or an S-oxide or S-dioxide thereof. Heteroaryl is, for example, furyl, thienyl (also known as thiophenyl), pyrrolyl, thiazolyl, isothiazolyl, pyrazolyl, oxazolyl, isoxazolyl, imidazolyl, [1,2,4]-triazolyl, pyridinyl, pyrimidinyl, pyrazinyl, indolyl, benzo[b]furyl (also known as benzfuryl), benz[b]thienyl (also known as benzthienyl or benzthiophenyl), indazolyl, benzimidazolyl, benztriazolyl, benzoxazolyl, benzthiazolyl, 1,2,3-benzothiadiazolyl, an imidazopyridinyl (such as imidazo[1,2a]pyridinyl), thieno[3,2-b]pyridin-6-yl, 1,2,3-benzoxadiazolyl (also known as benzo[1,2,3]thiadiazolyl), 2,1,3-benzothiadiazolyl, benzofurazan (also known as 2,1,3-benzoxadiazolyl), quinoxalinyl, a pyrazolopyridine (for example 1H-pyrazolo[3,4-b]pyridinyl), quinolinyl, isoquinolinyl, a naphthyridinyl (for example [1,6]naphthyridinyl or [1,8]naphthyridinyl), a benzothiazinyl or dibenzothiophenyl (also known as dibenzothienyl); or an N-oxide thereof, or an S-oxide or S-dioxide thereof.

Aryloxy includes phenoxy.

Heteroaryloxy includes pyridinyloxy and pyrimidinyloxy.

Phenyl(C₁₋₄ alkyl)alkyl is, for example, benzyl, 1-(phenyl)eth-1-yl or 1-(phenyl)eth-2-yl.

Heteroaryl(C₁₋₄ alkyl)alkyl is, for example, pyridinylmethyl, pyrimidinylmethyl or 1-(pyridinyl)eth-2-yl.

Phenyl(C₁₋₄ alkoxy) is, for example, benzyloxy or phenylCH(CH₃)O.

Heteroaryl(C₁₋₄ alkoxy) is, for example, pyridinylCH₂O, pyrimidinylCH₂O or pyridinylCH(CH₃)O.

Heteroaryl rings can carry various substituents including sulphonyl groups. A sulphonyl group on a heteroaryl ring can be a good leaving group (susceptible to nucleophilic displacement) and examples of such situation are: 2-methanesulphonyl-pyridine and 2- or 4-methanesulphonyl-pyrimidine. The present invention covers compounds including a heteroaryl ring carrying a sulphonyl group which are sufficiently stable (non-reactive) to be isolated using the experimental procedures described.

In one particular aspect the present invention provides a compound of formula (I) wherein: R⁴ is heterocyclyl optionally substituted by C₁₋₆ alkyl, C(O)H, C(O)(C₁₋₆ alkyl) or S(O)₂(C₁₋₆ alkyl); and a carbon atom of a heterocyclyl ring may also be substituted by halo (for example fluoro) or hydroxy.

In another aspect the present invention provides a compound of formula (I) wherein, unless specified otherwise aryl, phenyl and heteroaryl moieties are independently optionally substituted by one or more of halo, hydroxy, nitro, S(C₁₋₄ alkyl), S(O)(C₁₋₄ alkyl), S(O)₂(C₁₋₄ alkyl), S(O)₂NH₂, S(O)₂NH(C₁₋₄ alkyl), S(O)₂N(C₁₋₄ alkyl)₂, cyano, C₁₋₄ alkyl, C₁₋₄ alkoxy, C(O)NH₂, C(O)NH(C₁₋₄ alkyl), CO₂H, CO₂(C₁₋₄ alkyl), NHC(O)(C₁₋₄ alkyl), NHS(O)₂(C₁₋₄ alkyl), CF₃, CHF₂, CH₂F, CH₂CF₃ or OCF₃.

In a further aspect of the invention heteroaryl is pyrrolyl, thienyl, imidazolyl, thiazolyl, isoxazolyl, pyridinyl, pyrimidinyl, pyrazinyl or quinolinyl.

In another aspect of the invention R¹⁰, R¹³, R¹⁵, R¹⁶, and R¹⁸, are hydrogen or C₁₋₄ alkyl (for example methyl). In yet another aspect R¹⁰, R¹³, R¹⁵, R¹⁶ and R¹⁸ are hydrogen.

In a further aspect of the invention R¹¹, R¹², R¹⁴, R¹⁷, R¹⁸ and R¹⁹ are C₁₋₈ alkyl (optionally substituted by halo, C₁₋₆ alkoxy, C₁₋₆ haloalkoxy, C₃₋₆ cycloalkyl (optionally substituted by halo), C₅₋₆ cycloalkenyl, S(O)₂(C₁₋₄ alkyl), heteroaryl, phenyl, heteroaryloxy or aryloxy (for example phenoxy)), phenyl, heteroaryl, C₃₋₇ cycloalkyl (optionally substituted by halo or C₁₋₄ alkyl), C₄₋₇ cycloalkyl fused to a phenyl ring, C₅₋₇ cycloalkenyl, or, heterocyclyl (itself optionally substituted by oxo, C(O)(C₁₋₆ alkyl), S(O)ₖ(C₁₋₄ alkyl), halo or C₁₋₄ alkyl); k is 0, 1 or 2; or R¹⁰ and R¹¹, and/or R¹⁶ and R¹⁷ may join to form a 4-, 5- or 6-membered ring which optionally includes a nitrogen, oxygen or sulphur atom, said ring being optionally substituted by C₁₋₆ alkyl or C(O)(C₁₋₆ alkyl).

In yet another aspect of the invention R¹¹, R¹², R¹⁴, R¹⁷ and R¹⁹ are C₁₋₈ alkyl (optionally substituted by halo (such as fluoro)), phenyl (optionally substituted as recited above), C₃₋₆ cycloalkyl (optionally substituted by halo (such as fluoro)) or C-linked nitrogen containing heterocyclyl (optionally substituted on the ring nitrogen).

In another aspect of the invention R¹ is NR¹³C(O)R¹⁴, wherein R¹³ and R¹⁴ are as defined above.

In yet another aspect of the invention R¹⁴ is C₁₋₈ alkyl (optionally substituted by halo (such as fluoro, for example to form CF₃CH₂)), phenyl (optionally substituted as recited above), C₃₋₆ cycloalkyl (optionally substituted by halo (such as fluoro, for example to form 1,1-difluorocyclohex-4-yl)) or C-linked nitrogen containing heterocyclyl (such as pyran or piperidine, optionally substituted on the ring nitrogen).

In another aspect the present invention provides a compound of the invention wherein R¹⁴ is C₁₋₈ alkyl (optionally substituted by halo (such as fluoro, for example to form CF₃CH2)), phenyl (optionally substituted by halo) or C₅₋₆ cycloalkyl (optionally substituted by halo (such as fluoro, for example to form 1,1-difluorocyclohex-4-yl)).

In a further aspect of the invention heterocyclyl is optionally substituted (such as singly substituted for example on a ring nitrogen atom when present) by C₁₋₆ alkyl [optionally substituted by phenyl {which itself optionally substituted by halo, C₁₋₄ alkyl, C₁₋₄ alkoxy, cyano, nitro, CF₃, OCF₃, (C₁₋₄ alkyl)C(O)NH, S(O)₂NH₂, C₁₋₄ alkylthio or S(O)₂(C₁₋₄ alkyl)} or heteroaryl {which itself optionally substituted by halo, C₁₋₄ alkyl, C₁₋₄ alkoxy, cyano, nitro, CF₃, (C₁₋₄ alkyl)C(O)NH, S(O)₂NH₂, C₁₋₄ alkylthio or S(O)₂(C₁₋₄ alkyl)}], phenyl {optionally substituted by halo, C₁₋₄ alkyl, C₁₋₄ alkoxy, cyano, nitro, CF₃, OCF₃, (C₁₋₄ alkyl)C(O)NH, S(O)₂NTH₂, C₁₋₄ alkylthio or S(O)₂(C₁₋₄ alkyl)}, heteroaryl {optionally substituted by halo, C₁₋₄ alkyl, C₁₋₄ alkoxy, cyano, nitro, CF₃, (C₁₋₄ alkyl)C(O)NH, S(O)₂NH₂, C₁₋₄ alkylthio or S(O)₂(C₁₋₄ alkyl)}, S(O)₂NR⁴⁰R⁴¹, C(O)R⁴², C(O)NHR⁴³ or S(O)₂R⁴⁴; wherein R⁴⁰, R⁴¹, R⁴², R⁴³ and R⁴⁴ are, independently, hydrogen or C₁₋₆ alkyl.

In yet another aspect of the invention R¹ is optionally substituted aryl (such as optionally substituted phenyl) or optionally substituted heteroaryl, wherein the optional substituents are as recited above.

In another aspect of the invention R¹ is optionally substituted phenyl, wherein the optional substituents are as recited above.

In a further aspect of the invention R¹ is optionally substituted heterocyclyl, such as optionally substituted: piperidin-1-yl, piperidin-4-yl, piperazin-1-yl, pyrrolidin-1-yl, pyrrolidin-3-yl, azetidin-1-yl or azetidin-3-yl.

In a still further aspect of the invention the heterocyclyl of R¹ is mono-substituted by C₁₋₆ alkyl, C₃₋₇ cycloalkyl, phenyl {optionally substituted by halo (for example fluoro), C₁₋₄ alkyl (for example methyl), C₁₋₄ alkoxy (for example methoxy), CF₃ or OCF₃}, S(O)₂(C₁₋₄ alkyl) (for example S(O)₂CH₃, S(O)₂CH₂CH₃ or S(O)₂CH(CH₃)₂), S(O)₂(C₁₋₄ fluoroalkyl) (for example S(O)₂CF₃ or S(O)₂CH₂CF₃), S(O)₂phenyl {optionally substituted (such as mono-substituted) by halo (for example chloro), cyano, C₁₋₄ alkyl, C₁₋₄ alkoxy, CF₃, OCF₃, S(O)₂(C₁₋₄ alkyl) (for example S(O)₂CH₃ or S(O)₂CH₂CH₂CH₃) or S(O)₂(C₁₋₄ fluoroalkyl) (for example S(O)₂CH₂CF₃)}, benzyl {optionally substituted by halo (for example chloro or fluoro), C₁₋₄ alkyl, C₁₋₄ alkoxy (for example methoxy), CF₃ or OCF₃}, C(O)H, C(O)(C₁₋₄ alkyl), benzoyl {optionally substituted by halo (for example chloro or fluoro), C₁₋₄ alkyl (for example methyl), C₁₋₄ alkoxy, CF₃ or OCF₃}, C(O)₂(C₁₋₄ alkyl), C(O)NH₂, C(O)NH(C₁₋₄ alkyl) or C(O)NHphenyl {optionally substituted by halo (for example fluoro), C₁₋₄ alkyl, C₁₋₄ alkoxy, CF₃ or OCF₃}. Said heterocyclyl can also be mono-substituted by S(O)₂N(C₁₋₄ alkyl)₂. In a still further aspect when said heterocyclyl is a 4-substituted piperidin-1-yl, a 1-substituted piperidin-4-yl, a 4-substituted piperazin-1-yl, a 3-substituted pyrrolidin-1-yl, a 1-substituted pyrrolidin-3-yl, a 3-substituted azetidin-1-yl or a 1-substituted azetidin-3-yl (for example where said substituent is as recited earlier in this paragraph). In another aspect said heterocyclyl is a 1-substituted piperidin-4-yl or a 4-substituted piperazin-1-yl, wherein the substituent is S(O)₂(C₁₋₄ alkyl), S(O)₂(C₁₋₄ haloalkyl), S(O)₂(phenyl), S(O)₂N(C₁₋₄ alkyl)₂ or phenyl.

In a further aspect of the invention R¹ is phenyl mono-substituted by S(O)₂(C₁₋₄ alkyl), or piperidin-4-yl 1-substituted by S(O)₂(C₁₋₄ alkyl). The group S(O)₂(C₁₋₄ alkyl) is, for example, S(O)₂CH₃.

In yet another aspect of the invention R² is phenyl or heteroaryl, either of which is optionally substituted by halo, C₁₋₄ alkyl, C₁₋₄ alkoxy, S(O)ₚ(C₁₋₄ alkyl), nitro, cyano or CF₃; wherein p is 0, 1 or 2, for example 0 or 2. When R² is heteroaryl it is, for example an optionally substituted thiophenyl.

In a further aspect of the invention R² is phenyl optionally substituted by halo {such as fluoro or chloro (for example one or two fluoro)} or CF₃.

In a still further aspect R² is optionally substituted (for example unsubstituted or substituted in the 2-, 3-, or 3- and 5- positions) phenyl (such as optionally substituted by halo (such as chloro or fluoro), cyano, methyl, ethyl, methoxy, ethoxy or CF₃), or optionally substituted (for example unsubstituted or mono-substituted) heteroaryl (such as optionally substituted by halo (such as chloro or fluoro), cyano, methyl, ethyl, methoxy, ethoxy or CF₃).

In another aspect the invention provides a compound of the invention wherein R² is optionally substituted (for example unsubstituted or substituted in the 2-, 3-, or 3- and 5-positions) phenyl (such as optionally substituted by halo (for example chloro or fluoro)). In yet another aspect the invention provides a compound of the invention wherein R² is phenyl, 3-fluorophenyl, 3-chlorophenyl, 3-trifluoromethylphenyl, 3-chloro-5-fluorophenyl or 3,5-difluorophenyl. In a further aspect the invention provides a compound of the invention wherein R² is phenyl, 3-fluorophenyl, 3-chlorophenyl or 3,5-difluorophenyl. In a still further aspect the invention provides a compound of the invention wherein R² is 3,5-difluorophenyl.

In yet another aspect of the invention R³ is hydrogen or methyl. In a further aspect of the invention when R³ is C₁₋₄ alkyl (such as methyl) and the carbon to which R³ is attached has the R absolute configuration. In yet another aspect of the invention R³ is hydrogen.

In a further aspect the present invention provides a compound of the invention wherein n is 2.

In a still further aspect the invention provides a compound of the invention wherein A is absent.

In a further aspect the present invention provides a compound of formula (Ia): 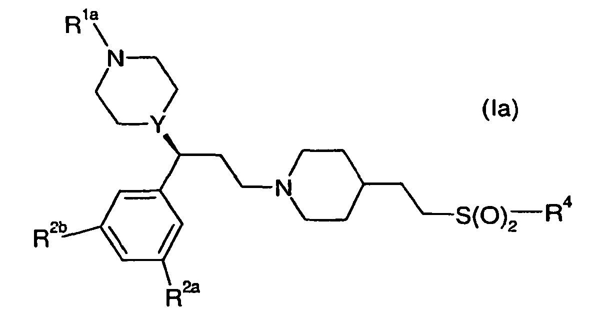 wherein Y is CH or N; R^{1a} is mono-substituted by C₁₋₆ alkyl, C₃₋₇ cycloalkyl, phenyl {optionally substituted by halo (for example fluoro), C₁₋₄ alkyl (for example methyl), C₁₋₄ alkoxy (for example methoxy), CF₃ or OCF₃}, S(O)₂(C₁₋₄ alkyl) (for example S(O)₂CH₃, S(O)₂CH₂CH₃ or S(O)₂CH(CH₃)₂), S(O)₂(C₁₋₄ fluoroalkyl) (for example S(O)₂CF₃ or S(O)₂CH₂CF₃), S(O)₂phenyl {optionally substituted (such as mono-substituted) by halo (for example chloro), cyano, C₁₋₄ alkyl, C₁₋₄ alkoxy, CF₃, OCF₃, S(O)₂(C₁₋₄ alkyl) (for example S(O)₂CH₃ or S(O)₂CH₂CH₂CH₃) or S(O)₂(C₁₋₄ fluoroalkyl) (for example S(O)₂CH₂CF₃)}, benzyl {optionally substituted by halo (for example chloro or fluoro), C₁₋₄ alkyl, C₁₋₄ alkoxy (for example methoxy), CF₃ or OCF₃}, C(O)H, C(O)(C₁₋₄ alkyl), benzoyl {optionally substituted by halo (for example chloro or fluoro), C₁₋₄ alkyl (for example methyl), C₁₋₄ alkoxy, CF₃ or OCF₃}, C(O)₂(C₁₋₄ alkyl), C(O)NH₂, C(O)NH(C₁₋₄ alkyl), C(O)NHphenyl {optionally substituted by halo (for example fluoro), C₁₋₄ alkyl, C₁₋₄ alkoxy, CF₃ or OCF₃} or S(O)₂N(C₁₋₄ alkyl)₂; R^{2a} and R^{2b} are, independently, hydrogen or halo (for example fluoro); and R⁴ is heterocyclyl optionally substituted by C₁₋₆ alkyl, C(O)H, C(O)(C₁₋₆ alkyl) or S(O)₂(C₁₋₆ alkyl); and a carbon atom of a heterocyclyl ring may also be substituted by halo (for example fluoro) or hydroxy. In another aspect of the invention R^{1a} is S(O)₂(C₁₋₄ alkyl). In yet another aspect of the invention Y is CH.

In a further aspect the present invention provides a compound of formula (Ib): 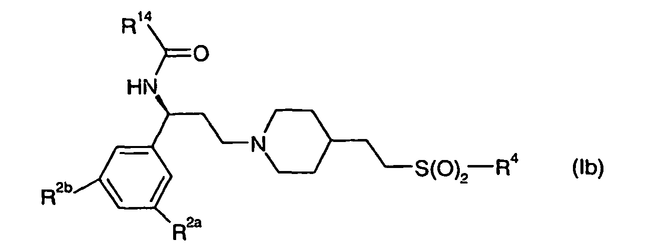 wherein R^{2a}, R^{2b}, R¹⁴ and R⁴ are as defined above.

In a still further aspect the present invention provides a compound of formula (Ic): 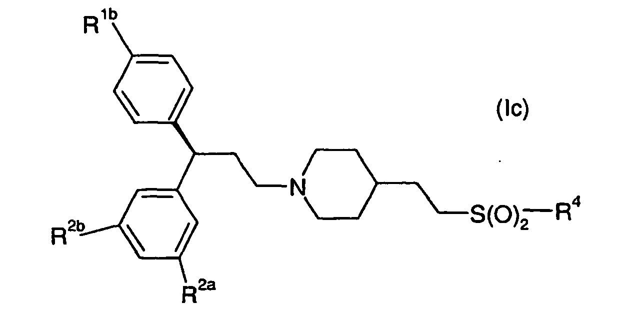 wherein R^{1b} is halo, hydroxy, nitro, S(C₁₋₄ alkyl), S(O)(C₁₋₄ alkyl), S(O)₂(C₁₋₄ alkyl), S(O)₂NH₂, S(O)₂NH(C₁₋₄ alkyl), S(O)₂N(C₁₋₄ alkyl)₂, cyano, C₁₋₄ alkyl, C₁₋₄ alkoxy, C(O)NH₂, C(O)NH(C₁₋₄ alkyl), CO₂H, CO₂(C₁₋₄ alkyl), NHC(O)(C₁₋₄ alkyl), NHS(O)₂(C₁₋₄ alkyl), CF₃, CHF₂, CH₂F, CH₂CF₃ or OCF₃; and R^{2a}, R^{2b} and R⁴ are as defined above. In another aspect of the invention R^{1b} is S(O)₂(C₁₋₄ alkyl).

In yet another aspect of the invention there is provided a compound of formula (Ia), (Ib) or (Ic) wherein R^{2a} and R^{2b} are, independently, hydrogen, halo (such as chloro or fluoro) or CF₃.

In a further aspect of the invention R⁴ is heterocyclyl optionally substituted by oxo, halogen, cyano, hydroxy, C₁₋₆ alkyl (itself optionally substituted by halogen, hydroxy, cyano or C₁₋₄ alkoxy), C₂₋₄ alkenyl, CO₂(C₁₋₄ alkyl), S(O)₂(C₁₋₄ alkyl), CH(O), S(O)₂(C₁₋₄ haloalkyl), C(O)(C₁₋₄ alkyl), C(O)(C₃₋₆ cycloalkyl), N(C₁₋₄ alkyl)₂, C(O)NH₂, C(O)N(C₁₋₄ alkyl)₂ or NHC(O)(C₁₋₄ alkyl). Heterocyclyl is, for example, piperidinyl, homopiperazinyl, thiomorpholinyl, pyrrolidinyl, piperazinyl, 1,2,3,6-tetrahydropyridinyl, morpholinyl, 2,5-dihydropyrrolyl, azetidinyl, 1,4-oxepanyl, 3-azabicyclo[3.2.1]octan-3-yl, 8-azaspiro[4.5]decanyl or 3-azabicyclo[3.1.0]hex-3-yl.

The compounds listed in Tables I, II and III illustrate the invention.

**Table I**

| Table I comprises compounds of formula (Ia) | | | | | | |
|---|---|---|---|---|---|---|
| 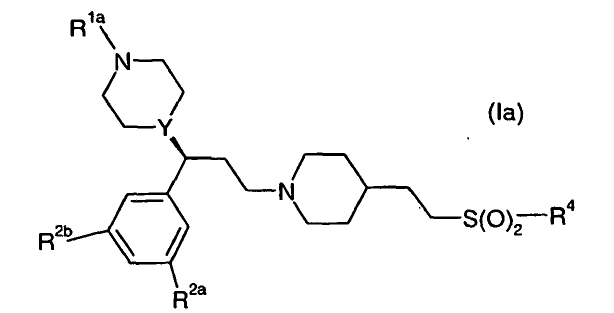 | | | | | | |

| Compound No | R^{1a} | R^{2a} | R^{2b} | R⁴ | Y | MS (MH⁺) |
|---|---|---|---|---|---|---|
| 1 | S(O)₂CH₃ | F | F | Piperidin-1-yl | CH | |
| 2 | S(O)₂CH₃ | F | F | Piperidin-4-yl | CH | 576 |
| 3 | S(O)₂CH₃ | F | F | N-trifluoromethanesulphonylpiperidin-4-yl | CH | 708 |
| 4 | S(O)₂CH₃ | F | F | N-acetylpiperidin-4-yl | CH | 618 |
| 5 | S(O)₂CH₃ | F | F | N-methanesulphonylpiperidin-4-yl | CH | 654 |
| 6 | S(O)₂CH₃ | F | F | 4-ethylpiperazin-1-yl | CH | |

**Table II**

| Table II comprises compounds of formula (Ib) | | | | |
|---|---|---|---|---|
| 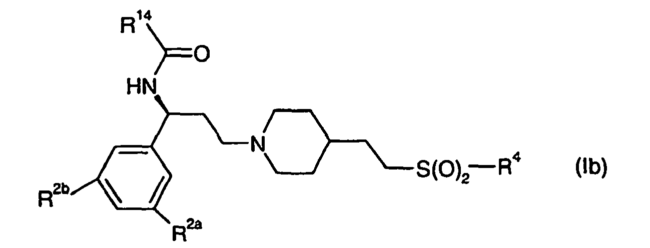 | | | | |

| Compound No | R¹⁴ | R^{2a} | R^{2b} | R⁴ |
|---|---|---|---|---|
| 1 | 3,3,3-Trifluoropropyl | F | F | Piperidin-1-yl |

**Table III**

| Table III comprises compounds of formula (Ic) | | | | | |
|---|---|---|---|---|---|
| 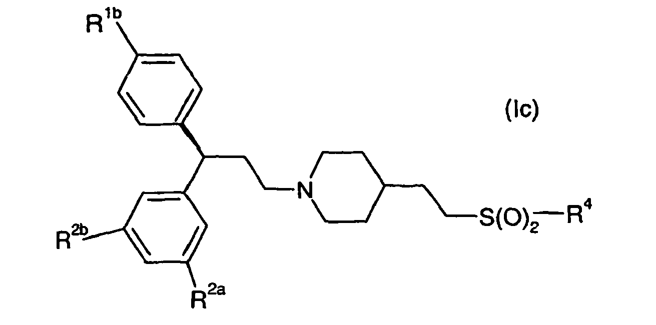 | | | | | |

| Compound No | R^{1b} | R^{2a} | R^{2b} | R⁴ | MS (MH+) |
|---|---|---|---|---|---|
| 1 | S(O)₂CH₃ | F | F | Piperidin-1-yl | 569 |
| 2 | S(O)₂CH₃ | F | F | N-acetylpiperidin-4-yl | 611 |
| 3 | S(O)₂CH₃ | F | F | N-CF₃S(O)₂-piperidin-4-yl | 701 |
| 4 | S(O)₂CH₃ | F | F | Piperidin-4-yl | 569 |
| 5 | S(O)₂CH₃ | F | F | N-CH₃S(O)₂-piperidin-4-yl | 649 |
| 6 | S(O)₂CH₃ | F | F | N-homopiperazinyl | 584 |
| 7 | S(O)₂CH₃ | F | F | 4-hydroxypiperidin-1-yl | 585 |
| 8 | S(O)₂CH₃ | F | F | N-thiomorpholinyl | 587 |
| 9 | S(O)₂CH₃ | F | F | 2-methylpyrrolidin-1-yl | 569 |
| 10 | S(O)₂CH₃ | F | F | N-piperazinyl | 570 |
| 11 | S(O)₂CH₃ | F | F | 1,2,3,6-tetrahydropyridin-1-yl | 567 |
| 12 | S(O)₂CH₃ | F | F | (3S)-pyrrolidin-1-yl-3-ol | 571 |
| 13 | S(O)₂CH₃ | F | F | N-morpholino | 571 |
| 14 | S(O)₂CH₃ | F | F | (3R)-3-fluoropyrrolidin-1-yl | 573 |
| 15 | S(O)₂CH₃ | F | F | pyrrolidin-1-yl | 555 |
| 16 | S(O)₂CH₃ | F | F | 2,5-dihydropyrrol-1-yl | 553 |
| 17 | S(O)₂CH₃ | F | F | N-azetidinyl | 541 |
| 18 | S(O)₂CH₃ | F | F | N-1,4-oxepanyl | 585 |
| 19 | S(O)₂CH₃ | F | F | 4-methylpiperazin-1-yl | 584 |
| 20 | S(O)₂CH₃ | F | F | (2S)-2-methylpiperazin-4-yl | |
| 21 | S(O)₂CH₃ | F | F | 4,4-difluoropiperidin-1-yl | 605 |
| 22 | S(O)₂CH₃ | F | F | 8-oxa-3-azabicyclo[3.2.1]octan-3-yl | 597 |
| 23 | S(O)₂CH₃ | F | F | (2S)-2-carboxamidopyrrolidin-1-yl | 598 |
| 24 | S(O)₂CH₃ | F | F | 4-formylpiperazin-1-yl | 598 |
| 25 | S(O)₂CH₃ | F | F | (2S)-2-methoxymethylpyrrolidin-1-yl | 599 |
| 26 | S(O)₂CH₃ | F | F | 4-hydroxymethylpiperidin-1-yl | 599 |
| 27 | S(O)₂CH₃ | F | F | (2R, 6S)-2,6-dimethylmorpholin-4-yl | 599 |
| 28 | S(O)₂CH₃ | F | F | 4-ethylpiperazin-1-yl | 598 |
| 29 | S(O)₂CH₃ | F | F | 2,6-dimethylpiperazin-4-yl | 598 |
| 30 | S(O)₂CH₃ | F | F | (3S)-N,N-dimethylaminopyrrolidin-1-yl | 598 |
| 31 | S(O)₂CH₃ | F | F | 4-(2-methoxyethyl)-piperazin-1-yl | 628 |
| 32 | S(O)₂CH₃ | F | F | (2R)-2-carboxamido-pyrrolidin-1-yl | 598 |
| 33 | S(O)₂CH₃ | F | F | (2R)-2-methoxymethylpyrrolidin-1-yl | 599 |
| 34 | S(O)₂CH₃ | F | F | (2R)-2-methylpiperazin-4-yl | 584 |
| 35 | S(O)₂CH₃ | F | F | 3-aminocarbonylpiperidin-1-yl | 612 |
| 36 | S(O)₂CH₃ | F | F | 3-acetylaminopyrrolidin-1-yl | 612 |
| 37 | S(O)₂CH₃ | F | F | 4-aminocarbonylpiperidin-1-yl | 612 |
| 38 | S(O)₂CH₃ | F | F | 4-(2-hydroxyethyl)-piperidin-1-yl | 613 |
| 39 | S(O)₂CH₃ | F | F | N-1,4-dioxa-8-azaspiro[4.5]decanyl | 627 |
| 40 | S(O)₂CH₃ | F | F | 3-hydroxyazetidin-1-yl | 557 |
| 41 | S(O)₂CH₃ | F | F | 4-(2-hydroxyethyl)-piperazin-1-yl | 614 |
| 42 | S(O)₂CH₃ | F | F | 4-methoxycarbonylpiperidin-1-yl | 627 |
| 43 | S(O)₂CH₃ | F | F | 4-acetylpiperazin-1-yl | 612 |
| 44 | S(O)₂CH₃ | F | F | 3-methanesulphonylpyrrolidin-1-yl | 633 |
| 45 | S(O)₂CH₃ | F | F | 4-acetyl-1,4-diazepan-1-yl | 626 |
| 46 | S(O)₂CH₃ | F | F | (2S)-2-N,N-dimethylaminocarbonylpyrroli din-1-yl | 626 |
| 47 | S(O)₂CH₃ | F | F | 3-(cyanomethyl)piperidin-1-yl | 608 |
| 48 | S(O)₂CH₃ | F | F | 4-(3-hydroxypropyl)-piperazin-1-yl | 628 |
| 49 | S(O)₂CH₃ | F | F | (3R)-3-hydroxypyrrolidin-1-yl | 571 |
| 50 | S(O)₂CH₃ | F | F | (3S)-3-fluoropyrrolidin-1-yl | 572 |
| 51 | S(O)₂CH₃ | F | F | (2S)-2-cyanopyrrolidin-1-yl | 580 |
| 52 | S(O)₂CH₃ | F | F | 4-ethoxycarbonylpiperazin-1-yl | 642 |
| 53 | S(O)₂CH₃ | F | F | 4-fluoropiperidin-1-yl | 587 |
| 54 | S(O)₂CH₃ | F | F | 4-(ethanesulphonyl)-piperazin-1-yl | 662 |
| 55 | S(O)₂CH₃ | F | F | 4-(cyclopropylcarbonyl)-piperazin-1-yl | 638 |
| 56 | S(O)₂CH₃ | F | F | 4-isopropylpiperazin-1-yl | 612 |
| 57 | S(O)₂CH₃ | F | F | 4-allyl-piperazin-1-yl | 610 |
| 58 | S(O)₂CH₃ | F | F | (lR,5S,6R)-6-hydroxymethyl-3-azabicyclo[3.1.0]hex-3-yl | 597 |
| 59 | S(O)₂CH₃ | F | F | 4-trifluoromethylpiperidin-1-yl | 637 |

In yet another aspect the invention provides each individual compound listed in the tables above.

The compounds of formula (I), (Ia), (Ib) and (Ic) are all compounds of the invention can be prepared as shown below. In the processes and Schemes presented it will be apparent to a person skilled in the art that if a reactive group (such as an NH group) is present then, for certain reactions, that group will need to be protected and subsequently deprotected. Also, it may be necessary to protect two reactive groups and then selectively deprotect. The use of protecting groups is described in Protective Groups in Organic Synthesis', 2nd edition, T.W. Greene & P.G.M. Wutz, Wiley-Interscience (1991).

A compound of the invention wherein R¹ is an N-linked optionally substituted heterocycle can be prepared by reacting a compound of formula (II): 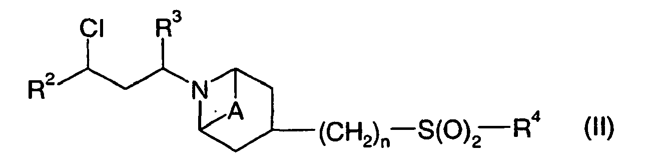 wherein R², R³, R⁴, n, A and X are as defined above, with a compound R¹H (wherein the H is on a heterocycle ring nitrogen atom) wherein R¹ is as defined above, in the presence of a suitable base (for example a tri(C₁₋₆ alkyl)amine such as triethylamine or Hunig's base), in a suitable solvent (such as a chlorinated solvent, for example dichloromethane) and, for example, at a room temperature (for example 10-30°C), optionally in the presence of sodium iodide.

A compound of the invention, wherein R³ is hydrogen, can be prepared by coupling a compound of formula (III): 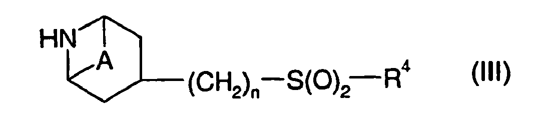 wherein R⁴, n, A and X are as defined above, with a compound of formula (IV): 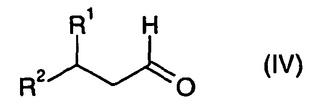 wherein R¹ and R² are as defined above, in the presence of NaBH(OAc)₃ (wherein Ac is C(O)CH₃) in a suitable solvent (such as a chlorinated solvent, for example dichloromethane) at room temperature (for example 10-30°C).

A compound of the invention, wherein R³ is hydrogen, can be prepared by coupling a compound of formula (III): 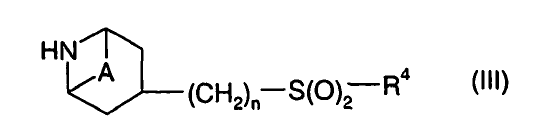 wherein R⁴, n, A and X are as defined above, with a compound of formula (V): 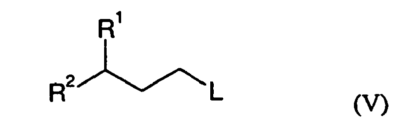 wherein R¹ and R² are as defined above and L is an activated leaving group such as halogen, tosylate, mesylate or triflate, in the presence of a base, such as potassium carbonate, in a suitable solvent (such as dioxane, acetonitrile or isopropanol) at a temperature from 60°C up to the boiling point of the solvent.

Alternatively, compounds of the invention can be prepared according to Schemes 1-7 (below). Note that in Scheme 6 the use of a homochiral diol will result in the synthesis of homochiral product.

Alternatively, compounds of the invention can be prepared by using or adapting methods described in WO01/87839, EP-A1-1013276, WO00/08013, WO99/38514, WO99/04794, WO00/76511, WO00/76512, WO00/76513, WO00/76514, WO00/76972 or US 2002/0094989.

The starting materials for these processes are either commercially available or can be prepared by literature methods, adapting literature methods or by following or adapting Methods herein described.

In a still further aspect the invention provides processes for preparing the compounds of formula (I), (Ia), (Ib) and (Ic). Many of the intermediates in the processes are novel and these are provided as further features of the invention.

The compounds of the invention have activity as pharmaceuticals, in particular as modulators (such as agonists, partial agonists, inverse agonists or antagonists) of chemokine receptor (such as CCR5) activity, and may be used in the treatment of autoimmune, inflammatory, proliferative or hyperproliferative diseases, or immunologically-mediated diseases (including rejection of transplanted organs or tissues and Acquired Immunodeficiency Syndrome (AIDS)).

The compounds of the present invention are also of value in inhibiting the entry of viruses (such as human immunodeficiency virus (HIV)) into target calls and, therefore, are of value in the prevention of infection by viruses (such as HIV), the treatment of infection by viruses (such as HIV) and the prevention and/or treatment of acquired immune deficiency syndrome (AIDS).

According to a further feature of the invention there is provided a compound of the formula (I), (Ia), (Ib) and (Ic), or a pharmaceutically acceptable salt thereof or a solvate thereof, for use in a method of treatment of a warm blooded animal (such as man) by therapy (including prophylaxis).

A method is disclosed for modulating chemokine receptor activity (such as CCR5 receptor activity) in a warm blooded animal, such as man, in need of such treatment, which comprises administering to said animal an effective amount of a compound of the present invention, or a pharmaceutically acceptable salt thereof or a solvate thereof.

The use of a compound of the formula (I), (Ia), (Ib) or (Ic), or a pharmaceutically acceptable salt thereof or a solvate thereof, as a medicament, is disclosed such as a medicament for the treatment of transplant rejection, respiratory disease, psoriasis or rheumatoid arthritis (such as rheumatoid arthritis). [Respiratory disease is, for example, COPD, asthma {such as bronchial, allergic, intrinsic, extrinsic or dust asthma, particularly chronic or inveterate asthma (for example late asthma or airways hyper-responsiveness)} or rhinitis {acute, allergic, atrophic rhinitis or chronic rhinitis including rhinitis caseosa, hypertrophic rhinitis, rhinitis purulenta, rhinitis sicca or rhinitis medicamentosa; membranous rhinitis including croupous, fibrinous or pseudomembranous rhinitis or scrofoulous rhinitis; seasonal rhinitis including rhinitis nervosa (hay fever) or vasomotor rhinitis}; and is particularly asthma or rhinitis].

In another aspect the present invention provides the use of a compound of the formula (I), (Ia), (Ib) or (Ic), or a pharmaceutically acceptable salt thereof or a solvate thereof, in the manufacture of a medicament for use in therapy (for example modulating chemokine receptor activity (such as CCR5 receptor activity (such as rheumatoid arthritis)) in a warm blooded animal, such as man).

The invention also provides a compound of the formula (I), (Ia), (Ib) or (Ic), or a pharmaceutically acceptable salt thereof or a solvate thereof, for use as a medicament, such as a medicament for the treatment of rheumatoid arthritis.

In another aspect the present invention provides the use of a compound of the formula (I), (Ia), (Ib) or (Ic), or a pharmaceutically acceptable salt thereof or a solvate thereof, in the manufacture of a medicament for use in therapy (for example modulating chemokine receptor activity (such as CCR5 receptor activity (such as rheumatoid arthritis)) in a warm blooded animal, such as man).

The invention further provides the use of a compound of formula (I), (Ia), (Ib) or (Ic), or a pharmaceutically acceptable salt thereof, in the manufacture of a medicament for use in the treatment of the following disease states:
(1) (the respiratory tract) obstructive diseases of airways including: chronic obstructive pulmonary disease (COPD) (such as irreversible COPD); asthma {such as bronchial, allergic, intrinsic, extrinsic or dust asthma, particularly chronic or inveterate asthma (for example late asthma or airways hyper-responsiveness)}; bronchitis {such as eosinophilic bronchitis}; acute, allergic, atrophic rhinitis or chronic rhinitis including rhinitis caseosa, hypertrophic rhinitis, rhinitis purulenta, rhinitis sicca or rhinitis medicamentosa; membranous rhinitis including croupous, fibrinous or pseudomembranous rhinitis or scrofoulous rhinitis; seasonal rhinitis including rhinitis nervosa (hay fever) or vasomotor rhinitis; sarcoidosis; farmer's lung and related diseases; nasal polyposis; fibroid lung or idiopathic interstitial pneumonia;
(2) (bone and joints) arthrides including rheumatic, infectious, autoimmune, seronegative spondyloarthropathies (such as ankylosing spondylitis, psoriatic arthritis or Reiter's disease), Behçet's disease, Sjogren's syndrome or systemic sclerosis;
(3) (skin and eyes) psoriasis, atopic dermatitis, contact dermatitis or other eczmatous dermitides, seborrhoetic dermatitis, Lichen planus, Phemphigus, bullous Phemphigus, Epidermolysis bullosa, urticaria, angiodermas, vasculitides erythemas, cutaneous eosinophilias, uveitis, Alopecia areata or vernal conjunctivitis;
(4) (gastrointestinal tract) Coeliac disease, proctitis, eosinophilic gastro-enteritis, mastocytosis, Crohn's disease, ulcerative colitis, irritable bowel disease or food-related allergies which have effects remote from the gut (for example migraine, rhinitis or eczema);
(5) (Allograft rejection) acute and chronic following, for example, transplantation of kidney, heart, liver, lung, bone marrow, skin or cornea; or chronic graft versus host disease; and/or
(6) (other tissues or diseases) Alzheimer's disease, multiple sclerosis, atherosclerosis, Acquired Immunodeficiency Syndrome (AIDS), Lupus disorders (such as lupus erythematosus or systemic lupus), erythematosus, Hashimoto's thyroiditis, myasthenia gravis, type I diabetes, nephrotic syndrome, eosinophilia fascitis, hyper IgE syndrome, leprosy (such as lepromatous leprosy), Peridontal disease, Sezary syndrome, idiopathic thrombocytopenia pupura or disorders of the menstrual cycle;
in a warm blooded animal, such as man.

A method of treating a chemokine mediated disease state (such as a CCR5 mediated disease state) in a warm blooded animal, such as man, is disclosed which comprises administering to a mammal in need of such treatment an effective amount of a compound of formula (I), (Ia), (Ib) or (Ic), or a pharmaceutically acceptable salt thereof or solvate thereof.

In order to use a compound of the invention, or a pharmaceutically acceptable salt thereof or solvate thereof, for the therapeutic treatment of a warm blooded animal, such as man, in particular modulating chemokine receptor (for example CCR5 receptor) activity, said ingredient is normally formulated in accordance with standard pharmaceutical practice as a pharmaceutical composition.

Therefore in another aspect the present invention provides a pharmaceutical composition which comprises a compound of the formula (I), (Ia), (Ib) or (Ic), or a pharmaceutically acceptable salt thereof or a solvate thereof (active ingredient), and a pharmaceutically acceptable adjuvant, diluent or carrier. In a further aspect the present invention provides a process for the preparation of said composition which comprises mixing active ingredient with a pharmaceutically acceptable adjuvant, diluent or carrier. Depending on the mode of administration, the pharmaceutical composition will, for example, comprise from 0.05 to 99 %w (per cent by weight), such as from 0.05 to 80 %w, for example from 0.10 to 70 %w, such as from 0.10 to 50 %w, of active ingredient, all percentages by weight being based on total composition.

The pharmaceutical compositions of this invention may be administered in standard manner for the disease condition that it is desired to treat, for example by topical (such as to the lung and/or airways or to the skin), oral, rectal or parenteral administration. For these purposes the compounds of this invention may be formulated by means known in the art into the form of, for example, aerosols, dry powder formulations, tablets, capsules, syrups, powders, granules, aqueous or oily solutions or suspensions, (lipid) emulsions, dispersible powders, suppositories, ointments, creams, drops and sterile injectable aqueous or oily solutions or suspensions.

A suitable pharmaceutical composition of this invention is one suitable for oral administration in unit dosage form, for example a tablet or capsule which contains between 0.1mg and 1g of active ingredient.

In another aspect a pharmaceutical composition of the invention is one suitable for intravenous, subcutaneous or intramuscular injection.

Each patient may receive, for example, an intravenous, subcutaneous or intramuscular dose of 0.01mgkg⁻¹ to 100mgkg⁻¹ of the compound, for example in the range of 0.1mgkg⁻¹ to 20mgkg⁻¹ of this invention, the composition being administered 1 to 4 times per day. The intravenous, subcutaneous and intramuscular dose may be given by means of a bolus injection. Alternatively the intravenous dose may be given by continuous infusion over a period of time. Alternatively each patient will receive a daily oral dose which is approximately equivalent to the daily parenteral dose, the composition being administered 1 to 4 times per day.

The following illustrate representative pharmaceutical dosage forms containing the compound of formula (I), (Ia), (Ib) or (Ic), or a pharmaceutically acceptable salt thereof or a solvent thereof (hereafter Compound X), for therapeutic or prophylactic use in humans:
(a)

| Tablet I | mg/tablet |
|---|---|
| Compound X | 100 |
| Lactose Ph.Eur. | 179 |
| Croscarmellose sodium | 12.0 |
| Polyvinylpyrrolidone | 6 |
| Magnesium stearate | 3.0 |

(b)

| Tablet II | mg/tablet |
|---|---|
| Compound X | 50 |
| Lactose Ph.Eur. | 229 |
| Croscarmellose sodium | 12.0 |
| Polyvinylpyrrolidone | 6 |
| Magnesium stearate | 3.0 |

(c)

| Tablet III | mg/tablet |
|---|---|
| Compound X | 1.0 |
| Lactose Ph.Eur. | 92 |
| Croscarmellose sodium | 4.0 |
| Polyvinylpyrrolidone | 2.0 |
| Magnesium stearate | 1.0 |

(d)

| Capsule | mg/capsule |
|---|---|
| Compound X | 10 |
| Lactose Ph.Eur. | 389 |
| Croscarmellose sodium | 100 |
| Magnesium stearate | 1.0 |

(e)

| Injection I | (50 mg/ml) |
|---|---|
| Compound X | 5.0% w/v |
| Isotonic aqueous solution | to 100% |

Buffers, pharmaceutically-acceptable cosolvents such as polyethylene glycol, polypropylene glycol, glycerol or ethanol or complexing agents such as hydroxy-propyl β-cyclodextrin may be used to aid formulation.

The above formulations may be obtained by conventional procedures well known in the pharmaceutical art. The tablets (a)-(c) may be enteric coated by conventional means, for example to provide a coating of cellulose acetate phthalate.

The invention further relates to combination therapies or compositions wherein a compound of formula (I), or a pharmaceutically acceptable salt, solvate or a solvate of a salt thereof, or a pharmaceutical composition comprising a compound of formula (I), or a pharmaceutically acceptable salt, solvate or a solvate of a salt thereof, is administered concurrently (possibly in the same composition) or sequentially with an agent for the treatment of any one of the above disease states.

In particular, for the treatment of the inflammatory diseases rheumatoid arthritis, psoriasis, inflammatory bowel disease, COPD, asthma and allergic rhinitis a compound of the invention can be combined with a TNF-α inhibitor (such as an anti-TNF monoclonal antibodie (such as Remicade, CDP-870 and D.sub2.E.sub7.), or a TNF receptor immunoglobulin molecule (such as Enbrel.reg.)), a non-selective COX-1 / COX-2 inhibitor (such as piroxicam or diclofenac; a propionic acid such as naproxen, flubiprofen, fenoprofen, ketoprofen or ibuprofen; a fenamate such as mefenamic acid, indomethacin, sulindac or apazone; a pyrazolone such as phenylbutazone; or a salicylate such as aspirin), a COX-2 inhibitor (such as meloxicam, celecoxib, rofecoxib, valdecoxib or etoricoxib) low dose methotrexate, lefunomide; ciclesonide; hydroxychloroquine, d-penicillamine or auranofin, or parenteral or oral gold.

The present invention still further relates to the combination of a compound of the invention together with:
- a leukotriene biosynthesis inhibitor, a 5-lipoxygenase (5-LO) inhibitor or a 5-lipoxygenase activating protein (FLAP) antagonist, such as zileuton, ABT-761, fenleuton, tepoxalin, Abbott-79175, Abbott-85761, an N-(5-substituted)-thiophene-2-alkylsulfonamide, a 2,6-di-tert-butylphenol hydrazones, a methoxytetrahydropyran such as Zeneca ZD-2138, SB-210661, a pyridinyl-substituted 2-cyanonaphthalene compound such as L-739,010; a 2-cyanoquinoline compound such as L-746,530; an indole or quinoline compound such as MK-591, MK-886 or BAY x 1005;
- a receptor antagonist for a leukotriene LTB.sub4., LTC.sub4., LTD.sub4. or LTE.sub4. selected from the group consisting of a phenothiazin-3-one such as L-651,392; an amidino compound such as CGS-25019c; a benzoxalamine such as ontazolast; a benzenecarboximidamide such as BILI. 284/260; or a compound such as zafirlukast, ablukast, montelukast, pranlukast, verlukast (MK-679), RG-12525, Ro-245913, iralukast (CGP 45715A) or BAY x 7195;
- a PDE4 inhibitor including an inhibitor of the isoform PDE4D;
- an antihistaminic H.sub1. receptor antagonist such as cetirizine, loratadine, desloratadine, fexofenadine, astemizole, azelastine or chlorpheniramine;
- a gastroprotective H.sub2. receptor antagonist;
- an α.sub1.- and α.sub2.-adrenoceptor agonist vasoconstrictor sympathomimetic agent, such as propylhexedrine, phenylephrine, phenylpropanolamine, pseudoephedrine, naphazoline hydrochloride, oxymetazoline hydrochloride, tetrahydrozoline hydrochloride, xylometazoline hydrochloride or ethylnorepinephrine hydrochloride;
- an anticholinergic agent such as ipratropium bromide, tiotropium bromide, oxitropium bromide, pirenzepine or telenzepine;
- a β.sub1.- to β.sub4.-adrenoceptor agonist such as metaproterenol, isoproterenol, isoprenaline, albuterol, salbutamol, formoterol, salmeterol, terbutaline, orciprenaline, bitolterol mesylate or pirbuterol, or a methylxanthanine including theophylline and aminophylline; sodium cromoglycate; or a muscarinic receptor (M1, M2, and M3) antagonist;
- an insulin-like growth factor type I (IGF-1) mimetic;
- an inhaled glucocorticoid with reduced systemic side effects, such as prednisone, prednisolone, flunisolide, triamcinolone acetonide, beclomethasone dipropionate, budesonide, fluticasone propionate or mometasone furoate;
- an inhibitor of a matrix metalloprotease (MMP), such as a stromelysin, a collagenase, or a gelatinase or aggrecanase; such as collagenase-1 (MMP-1), collagenase-2 (MMP-S), collagenase-3 (MMP-13), stromeIysin-1 (MMP-3), stromelysin-2 (MMP-10), and stromelysin-3 (MMP-11) or MMP-12;
- a modulator of chemokine receptor function such as CCR1, CCR2, CCR2A, CCR2B, CCR3, CCR4, CCR5, CCR6, CCR7, CCR8, CCR9, CCR10 and CCR11 (for the C-C family); CXCR1, CXCR2, CXCR3, CXCR4 and CXCR5 (for the C-X-C family) and CX₃CR1 for the C-X₃-C family;
- an osteoporosis agent such as roloxifene, droloxifene, lasofoxifene or fosomax;
- an immunosuppressant agent such as FK-506, rapamycin, cyclosporine, azathioprine or methotrexate; or,
- an existing therapeutic agent for the treatment of osteoarthritis, for example a non-steroidal anti-inflammatory agent (hereinafter NSAID's) such as piroxicam or diclofenac, a propionic acid such as naproxen, flubiprofen, fenoprofen, ketoprofen or ibuprofen, a fenamate such as mefenamic acid, indomethacin, sulindac or apazone, a pyrazolone such as phenylbutazone, a salicylate such as aspirin, a COX-2 inhibitor such as celecoxib, valdecoxib, rofecoxib or etoricoxib, an analgesic or intra-articular therapy such as a corticosteroid or a hyaluronic acid such as hyalgan or synvisc, or a P2X7 receptor antagonist.

The present invention still further relates to the combination of a compound of the invention together with: (i) a tryptase inhibitor; (ii) a platelet activating factor (PAF) antagonist; (iii) an interleukin converting enzyme (ICE) inhibitor; (iv) an IMPDH inhibitor; (v) an adhesion molecule inhibitor including a VLA-4 antagonist; (vi) a cathepsin; (vii) a MAP kinase inhibitor; (viii) a glucose-6 phosphate dehydrogenase inhibitor; (ix) a kinin-B.subl. - and B.sub2. -receptor antagonist; (x) an anti-gout agent, e.g., colchicine; (xi) a xanthine oxidase inhibitor, e.g., allopurinol; (xii) an uricosuric agent, e.g., probenecid, sulfinpyrazone or benzbromarone; (xiii) a growth hormone secretagogue; (xiv) a transforming growth factor (TGFβ); (xv) a platelet-derived growth factor (PDGF); (xvi) a fibroblast growth factor, e.g., basic fibroblast growth factor (bFGF); (xvii) a granulocyte macrophage colony stimulating factor (GM-CSF); (xviii) a capsaicin cream; (xix) a Tachykinin NK.sub1. and NK.sub3. receptor antagonist selected from the group consisting of NKP-608C; SB-233412 (talnetant); and D-4418; (xx) an elastase inhibitors selected from the group consisting of UT-77 and ZD-0892; (xxi) a TNFα converting enzyme inhibitor (TACE); (xxii) an induced nitric oxide synthase inhibitor (iNOS); or (xxiii) a chemoattractant receptor-homologous molecule expressed on TH2 cells (a CRTH2 antagonist).

The invention will now be illustrated by the following non-limiting Examples in which, unless stated otherwise:
(i) temperatures are given in degrees Celsius (°C); operations were carried out at room or ambient temperature, that is, at a temperature in the range of 18-25°C;
(ii) organic solutions were dried over anhydrous magnesium sulfate; evaporation of solvent was carried out using a rotary evaporator under reduced pressure (600-4000 Pascals; 4.5-30 mm Hg) with a bath temperature of up to 60°C;
(iii) chromatography unless otherwise stated means flash chromatography on silica gel; thin layer chromatography (TLC) was carried out on silica gel plates; where a "Bond Elut" column is referred to, this means a column containing 10g or 20g of silica of 40 micron particle size, the silica being contained in a 60ml disposable syringe and supported by a porous disc, obtained from Varian, Harbor City, California, USA under the name "Mega Bond Elut SI". Where an "Isolute^{™} SCX column" is referred to, this means a column containing benzenesulphonic acid (non-endcapped) obtained from International Sorbent Technology Ltd., 1st House, Duffryn Industial Estate, Ystrad Mynach, Hengoed, Mid Glamorgan, UK. Where "Argonaut^{™} PS-*tris*-amine scavenger resin" is referred to, this means a *tris*-(2-aminoethyl)amine polystyrene resin obtained from Argonaut Technologies Inc., 887 Industrial Road, Suite G, San Carlos, California, USA.
(iv) in general, the course of reactions was followed by TLC and reaction times are given for illustration only;
(v) yields, when given, are for illustration only and are not necessarily those which can be obtained by diligent process development; preparations were repeated if more material was required;
(vi) when given, ¹H NMR data is quoted and is in the form of delta values for major diagnostic protons, given in parts per million (ppm) relative to tetramethylsilane (TMS) as an internal standard, determined at 300 MHz using perdeuterio DMSO (CD₃SOCD₃) as the solvent unless otherwise stated; coupling constants (J) are given in Hz;
(vii) chemical symbols have their usual meanings; SI units and symbols are used;
(viii) solvent ratios are given in percentage by volume;
(ix) mass spectra (MS) were run with an electron energy of 70 electron volts in the chemical ionisation (APCI) mode using a direct exposure probe; where indicated ionisation was effected by electrospray (ES); where values for m/z are given, generally only ions which indicate the parent mass are reported, and unless otherwise stated the mass ion quoted is the positive mass ion - (M+H)⁺;
(x) LCMS characterisation was performed using a pair of Gilson 306 pumps with Gilson 233 XL sampler and Waters ZMD4000 mass spectrometer. The LC comprised water symmetry 4.6x50 column C18 with 5 micron particle size. The eluents were: A, water with 0.05% formic acid and B, acetonitrile with 0.05% formic acid. The eluent gradient went from 95% A to 95% B in 6 minutes. Where indicated ionisation was effected by electrospray (ES); where values for m/z are given, generally only ions which indicate the parent mass are reported, and unless otherwise stated the mass ion quoted is the positive mass ion - (M+H)⁺;
(xi) PS-NCO resin is an isocyanate resin and is available from Argonaut; and,
(xii) the following abbreviations are used:
   - THF: tetrahydrofuran;
   - Boc: tert-butoxycarbonyl
   - DMF: N,N-dimethylformamide
   - DCM: dichloromethane
   - DIPEA: N,N-Diisopropylethylamine
   - R-BINAP: R 2,2'-Bis(diphenylphosphino)-1,1'-binaphthyl

### Example 1

This Example illustrates the preparation of (*R*)-1-{3-(3,5-difluorophenyl)-3-[4-(methylsulfonyl)phenyl]propyl}-4-[2-(piperidin-1-ylsulfonyl)ethyl]piperidine hydrochloride (Compound No. 1 of Table III].

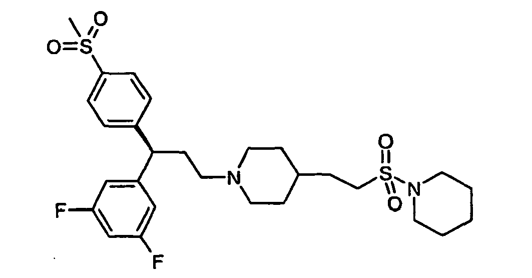

A mixture of 1-[(2-piperidin-4-ylethyl)sulfonyl]piperidine hydrochloride (Method B, 400mg, 1.35mmol), (*R*)-3-(3,5-difluorophenyl)-3-[4-(methylsulfonyl)phenyl]propyl 4-methylbenzenesulfonate (Method C, 600mg, 1.25mmol) and potassium carbonate (500mg, 3.62mmol) in acetonitrile (10mL) was heated to reflux for 6h. The mixture was allowed to cool then evaporated and the residue partitioned between ethyl acetate and water. The organic phase was evaporated and the residue purified by silica column chromatography. The crude product was treated with a solution of hydrogen chloride in methanol to yield the title compound as a solid (330mg); NMR: 1.4-1.6 (m, 11H), 1.85 (m, 2H), 2.6 (m, 2H), 2.8 (m, 4H), 3.0 (m, 2H), 3.1 (m, 4H), 3.15 (s, 3H), 3.5 (m, 2H), 4.25 (dd, 1H), 7.05 (m, 1H), 7.15 (d, 2H), 7.65 (d, 2H). 7.85 (d, 2H); LC-MS: 569.

### Example 2

This Example describes the preparation of 1-{[2-(1-{(3*R*)-3-(3,5-difluorophenyl)-3-[4-(methylsulfonyl)phenyl]propyl}piperidin-4-yl)ethyl]sulfonyl}-4-ethylpiperazine (Compound 28 Table III).

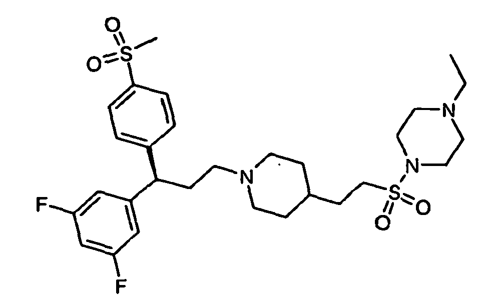

N-Ethylpiperazine (0.2g) was added to a stirred solution of 2-(1-{ (3R)-3-(3,5-difluorophenyl)-3-[4-(methylsulfonyl)phenyl]propyl}-4-piperidinyl)ethanesulfonyl chloride (0.26g) (Method I) in dichloromethane (20ml) at ambient temperature under an argon atmosphere. The reaction mixture was allowed to stir at ambient temperature for 16 hours before washing with brine (20ml), drying over magnesium sulphate, filtering and evaporating to a yellow oil. This residue was purified by chromatography on a 20g silica isolute cartridge, eluting with 0-10% methanol - dichloromethane. The recovered material was then further purified by chromatography on a 10g NH₂ isolute cartridge using dichloromethane as eluent. Evaporation of solvent gave the title compound as a white foam, yield (0.115g).
NMR(CDCl₃): 1.08 (t, 3H), 1.3 (m, 3H), 1.633 (m, 2H), 1.76 (m, 2H), 1.885 (m, 2H), 2.2 (m, 4H), 2.46 (m, 2H), 2.5 (t, 4H), 2.8 (m, 2H), 2.9 (m, 2H), 3.03 (s, 3H), 3.30 (t, 4H), 4.11 (m, 1H), 6.66 (m, 1H), 6.74 (m, 2H), 7.41 (d, 2H), 7.875 (d, 2H); MS: 598.25 (MH)⁺.

### Example 3

This Example describes the preparation of 1-{(3*R*)-3-(3,5-difluorophenyl)-3-[4-(methylsulfonyl)phenyl]propyl }-4-[2-(piperidin-4-ylsulfonyl)ethyl]piperidine (Compound 4 Table III).

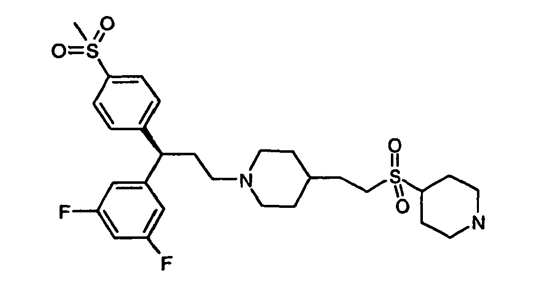

A solution of benzyl 4-{[2-(1-{(3*R*)-3-(3,5-difluorophenyl)-3-[4-(methylsulfonyl)phenyl]propyl}piperidin-4-yl)ethyl]sulfonyl}piperidine-1-carboxylate (1.52 g) in ethanol (22 ml) containing 20% Pd(OH)₂ (152 mg) on carbon as catalyst was hydrogenated. The reaction mixture was filtered through Celite® and the filter cake was washed with methanol (100 ml). The combined organics were evaporated to dryness to give the title compound, LC-MS MH⁺ 569. NMR (DMSO-d₆): 1.17 (2H, m), 1.32 (1H, m),1.60-1.81 (6H, m), 1.91 (2H, m), 2.10 (2H, d), 2.24 (4H, m), 2.81 (4H, m), 3.07 (2H, m), 3.17 (3H, s), 3.31 (3H, m), 4.18 (1H, t), 7.02(1H, t), 7.12 (2H, d), 7.63 (2H, d), 7.84 (2H, d).

Using the method described in above but with benzyl 4-{[2-(1-{(3*R*)-3-(3,5-difluorophenyl)-3-[1-(methylsulfonyl)piperidin-4-yl]propyl}piperidin-4-yl)ethyl]sulfonyl}piperidine-1-carboxylate as starting material [prepared using (3R)-3-(3,5-difluorophenyl)-3-[1-(methylsulfonyl)piperidin-4-yl]propanal as starting material according to Method G, followed by Dess-Martin oxidation as described in Method F, step 7] there is obtained 1-{(3*R*)-3-(3,5-difluorophenyl)-3-[1-(methylsulfonyl)piperidin-4-yl]propyl}-4-[2-(piperidin-4-ylsulfonyl)ethyl]piperidine (Compound 2 Table I), LC-MS MH⁺ 576. NMR (CDCl₃) (Part spectrum described) 1.18-2.96 (31H, m), 2.72 (3H, s), 3.22 (2H, d), 3.70 (2H, m). 3.82 (1H, d), 6.64 (3H,m).

### Example 4

This Example describes the preparation of 1-acetyl-4-{[2-(1-{(3*R*)-3-(3,5-difluorophenyl)-3-[4-(methylsulfonyl)phenyl]propyl}piperidin-4-yl)ethyl]sulfonyl}piperidine (Compound 2 Table III).

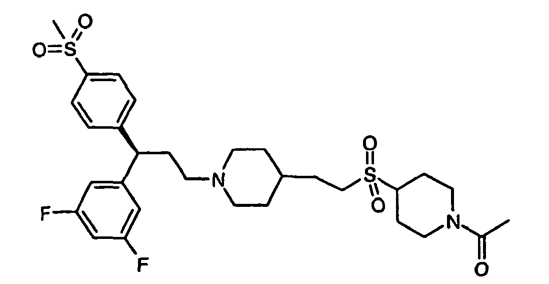

Acetic anhydride (50 µl) was added to a mixture of 1-{(3*R*)-3-(3,5-difluorophenyl)-3-[4-(methylsulfonyl)phenyl]propyl}-4-[2-(pipexidin-4-ylsulfonyl)ethyl]piperidine (200 mg) (Example 3) and MP-carbonate (514 mg) in dichloromethane (3.5 ml) at 0 °C under an atmosphere of argon with stirring. The mixture was allowed to warm to room temperature and was stirred for 18 hours. The resin was filtered and washed with 10% methanol in dichloromethane (10 ml). The combined organics were evaporated to dryness and passed down an Isolute 20g silica column eluting with a mixture of methanol and ethyl acetate (1:9). The title compound was obtained as a white foam, yield 79 mg, LC-MS MH⁺ 611. LC-MS MH⁺ 611. NMR (CDCl₃): 1.21-1.43 (3H, m), 1.64-1.93 (8H, m), 2.03-2.26 (5H, m), 2.10 (3H. s), 2.61 (1H, t), 2.81-2.96 (5H, m), 3.07 (3H, s), 3.11 (2H, m), 3.98 (1H, m), 4.09 (1H, m), 4.77 (1H, m), 6.65 (1H, m), 6.72 (2H, d), 7.38 (2H, d), 7.86 (2H, d).

Following the method described above and using 1-{(3*R*)-3-(3,5-difluorophenyl)-3-[1-(methylsulfonyl)piperidin-4-yl]propyl}-4-[2-(piperidin-4-ylsulfonyl)ethyl]piperidine as starting material there is obtained 1-acetyl-4-{[2-(1-{(3*R*)-3-(3,5-difluorophenyl)-3-[1-(methylsulfonyl)piperidin-4-yl]propyl}piperidin-4-yl)ethyl]sulfonyl}piperidine (Compound 4 Table I) as a white foam, LC-MS MH⁺ 618. NMR (CDCl₃) 1.14-2.20 (22H, m), 2.08 (3H, s), 2.40 (1H, t), 2.54 (1H, t), 2.62 (2H, m), 2.72 (3H, s), 2.88 (4H, m), 3.08 (2H, m), 3.70 (1H, m), 3.84 (1H, m) 4.00 (1H, m), 4.78 (1H, m), 6.64 (3H, m).

### Example 5

This Example describes the preparation of 1-{(3*R*)-3-(3,5-difluorophenyl)-3-[1-(methylsulfonyl)piperidin-4-yl]propyl}-4-[2-({1-[(trifluoromethyl)sulfonyl]piperidin-4-yl}sulfonyl)ethyl]piperidine (Compound 3 Table I)

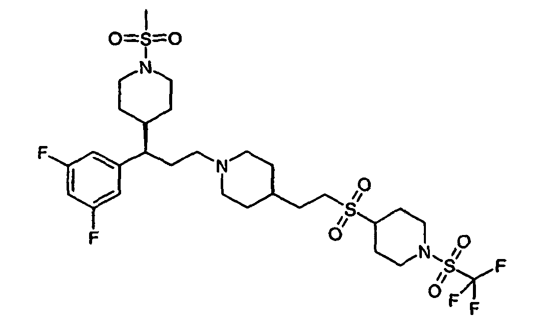

To a stirred solution of 1-{(3*R*)-3-(3,5-difluorophenyl)-3-[1-(methylsulfonyl)piperidin-4-yl]propyl}-4-[2-(piperidin-4-ylsulfonyl)ethyl]piperidine (173mg, 0.30mmol) in dichloromethane (3ml) under an atmosphere of Argon at 0 °C was added triethylamine (83µl, 0.6mmol) and then trifluoromethanesulfonic anhydride (66µl, 0.39mmol). The mixture was allowed to warm to room temperature and then stirred for 2 hours, diluted with dichloromethane (50ml) and washed with ammonium chloride solution (2 ×25ml), brine (25ml), dried and then evaporated to dryness. The residue was passed down an Isolute 20g silica column eluting with a solvent gradient of 5% methanol/ethyl acetate to 8% methanol/ethyl acetate. The product was obtained as a white foam, yield 63 mg. LC-MS MH⁺ 708. NMR (CDCl₃) 1.18-2.32 (22H, m), 2.42 (1H, t), 2.50 (1H, t), 2.62 (1H, t), 2.74 (3H, s), 2.90-3.20 (7H, m), 3.70 (1H, d), 3.82 (1H, d), 4.08 (2H, d), 6.64 (3H, m).

Following the method described above and using 1-{(3R)-3-(3,5-difluorophenyl)-3-[4-(methylsulfonyl)phenyl]propyl}-4-[2-(piperidin-4-ylsulfonyl)ethyl]piperidine (Example 2) as starting material there is obtained 1-{(3*R*)-3-(3,5-difluorophenyl)-3-[4-(methylsulfonyl)phenyl]propyl}-4-[2-({1-[(trifluoromethyl)sulfonyl]piperidin-4-yl}sulfonyl)ethyl]piperidine (Compound 3 Table III) as a white foam 75mg. LC-MS MH⁺ 701. NMR (CDCl₃) 1.56-2.60 (17H, m), 2.92-3.20 (7H, m), 3.02 (3H, s), 4.10 (3H, m), 6.68 (1H, t), 6.76 (2H, d), 7.44 (2H, d), 7.86 (2H, d).

### Example 6

This Example describes the preparation of 1-{(3*R*)-3-(3,5-difluorophenyl)-3-[1-(methylsulfonyl)piperidin-4-yl]propyl}-4-(2-{[1-(methylsulfonyl)piperidin-4-yl]sulfonyl}ethyl)piperidine (Compound 5 Table I).

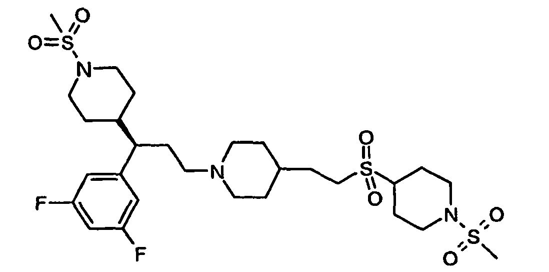

To a stirred solution of 1-{(3*R*)-3-(3,5-difluorophenyl)-3-[1-(methylsulfonyl)piperidin-4-yl]propyl}-4-[2-(piperidin-4-ylsulfonyl)ethyl]piperidine (173mg, 0.30mmol) in dichloromethane (3ml) under an atmosphere of Argon at 0 °C was added triethylamine (83µl, 0.6mmol) and then methanesulfonyl chloride (36µl, 0.45mmol). The mixture was allowed to warm to room temperature and then stirred for 2 hours, diluted with dichloromethane (50ml) and washed with ammonium chloride solution (2 x 25ml), brine (25ml), dried and then evaporated to dryness. The residue was passed down an Isolute 20g silica column eluting with a mixture of methanol and ethyl acetate (1:4). The product was obtained as a white foam, yield 88 mg. LC-MS MH⁺ 654. NMR (CDCl₃) 1.14-2.58 (26H, m), 2.60 (1H, t), 2.74 (3H, s), 2.80 (2H, m), 2.80 (3H, s), 2.94 (3H, m), 3.72 (1H, d), 3.84 (1H, d), 3.96 (2H, d), 6.68 (3H, m).

Following the method described above and using 1-{(3*R*)-3-(3,5-difluorophenyl)-3-[4-(methylsulfonyl)phenyl]propyl}-4-[2-(piperidin-4-ylsulfonyl)ethyl]piperidine (Example 2) as starting material there is obtained 1-{(3*R*)-3-(3,5-difluorophenyl)-3-[4-(methylsulfonyl)phenyl]propyl }-4-(2-{[1-(methylsulfonyl)piperidin-4-yl]sulfonyl}ethyl)piperidine (Compound 5 Table III) as a white foam 75mg. LC-MS MH⁺ 649. NMR (CDCl₃) 1.22-1.44 (3H, m), 1.68 (2H, d), 1.80 (2H, m), 1.86-2.02 (5H, m), 2.22 (5H, m), 2.78-2.98 (7H, m), 2.82 (3H, s), 3.04 (3H, s), 3.94 (2H, m), 4.10 (1H, m) 6.66 (1H, t), 6.74 (2H, d), 7.42 (2H, d), 7.86 (2H, d).

### Method A

### (S)-3-Phenyl-3-(4-methanesulfonylphenyl)propionaldehyde

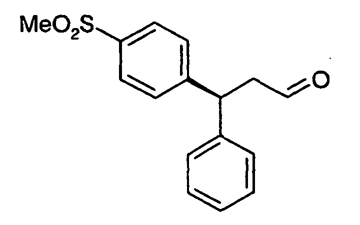

### Step 1: Preparation of E-(4S,5R)-1-(3-[4-methanesulphonylphenyl]acryloyl)-3,4-dimethyl-5-phenyl-imidazotidin-2-one

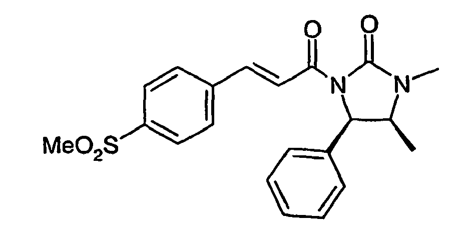

To a stirred solution of 3-(4-methanesulphonylphenyl)acrylic acid (7.14g, 31.5mmol) in DCM (10mL) was added thionyl chloride (3mL, 34.7mmol) dropwise and the resulting mixture was stirred at room temperature for 18h. To this solution was added DIPEA (5.04mL, 28.9mmol) dropwise at room temperature. The resulting solution was added to a stirred solution of (4*R*, 5*S*)-1,5-dimethyl-4-phenyl-imidazolidin-2-one (5.0g, 26.3mmol) in DCM (20mL) and DIPEA (4.58mL, 26.9mmol) and the resulting mixture stirred at room temperature for 4h. The mixture was washed with water and brine, pre-absorbed onto a Bond Elut and eluted with a gradient of isohexane to ethyl acetate giving the title compound as a solid (7.61g, 73%); NMR (CDCl₃): 0.84 (d, 3H), 2.89 (s, 3H), 3.04 (s, 3H), 3.98 (m, 1H), 5.42 (d, 1H), 7.20 (m, 2H), 7.32 (m, 3H), 7.69 (d, 1H), 7.74 (d, 2H), 7.93 (d, 2H), 8.31 (d, 1H); MS: 399.

### Step 2: Preparation of (4S, 5R)-1-[(S)-3-(4-methanesulfonyl-phenyl)-3-phenyl-propionyl]-3,4-dimethyl-5-phenyl-imidazolidin-2-one

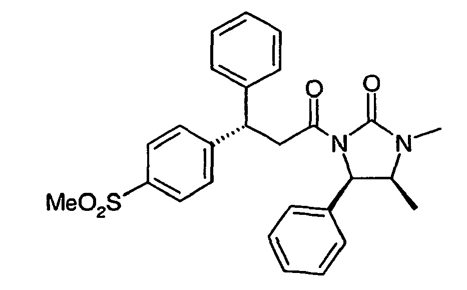

To a mixture of copper (I) iodide (960mg, 5.0mmol) and THF (20mL) was added *N*,*N*,*N'*,*N'*-tetramethylethylenediamine (0.83mL, 5.5mmol) and the resulting mixture was stirred at room temperature for 10min. then cooled to -78°C. Phenylmagnesium bromide (5.0mL, 1M in THF, 5.0mmol) was added and the resulting mixture stirred at -78°C for 15min. A solution of di-n-butylboron triflate (3.0mL, 1M in diethyl ether, 3.0mmol) and (*E*)-(4*S*, 5*R*)-1-(3-[4-methanesulfonylphenyl]acryloyl)-3,4-dimethyl-5-phenyl-imidazolidin-2-one (1.0g, 2.51mmol) in THF (15mL) was added and the resulting mixture was stirred whilst allowing to warm to room temperature for 18h. The reaction mixture was washed with saturated aqueous ammonium chloride, water and brine, dried (MgSO₄) and evaporated. The residue was purified by eluting through a 20g Bond Elut with gradient of isohexane to ethyl acetate giving the sub-titled compound (1.49g, 100%); NMR (CDCl₃): 0.78 (d, 3H), 2.82 (s, 3H), 3.00 (s, 3H), 3.78 (dd, 1H), 3.80 (m, 1H), 3.98 (dd, 1H), 4.72 (m, 1H), 5.19 (d, 1H), 6.99 (m, 2H), 7.22 (m, 8H), 7.48 (d, 2H), 7.79 (d, 2H); MS: 477.

### Step 3: Preparation of (S)-3-phenyl-3-(4-methanesulphonylphenyl)propan-1-ol

To a solution of (4*S*, 5*R*)-1-[(*S*)-3-(4-methanesulphonyl-phenyl)-3-phenyl-propionyl]-3,4-dimethyl-5-phenyl-imidazolidin-2-one (846mg, 1.78mmol) in THF (20mL) at 0°C was added lithium aluminium hydride (3.6mL, 1M in THF, 3.6mmol) and the resulting mixture was stirred for l5min. The reaction was quenched by the addition of 2M aqueous sodium hydroxide. The phases were separated and the organic phase pre-absorbed onto a Bond Elut and eluted with a gradient of isohexane to ethyl acetate giving the sub-titled compound as a white solid (285mg, 55%); NMR (CDCl₃): 1.63 (br s, 1H), 2.33 (m, 2H), 3.00 (s, 3H), 3.59 (t, 2H), 4.28 (t, 1H), 7.23 (m, 5H), 7.43 (d, 2H), 7.82 (d, 2H).

### Step 4: Preparation of the title compound

To a solution of (*S*)-3-phenyl-3-(4-methanesulfonylphenyl)propan-1-ol (244mg, 0.84mmol) in DCM (5mL) was added Dess-Martin periodinane (392mg, 0.92mmol) and the resulting mixture was stirred at room temperature for 1.5h. The mixture was washed with 2M aqueous sodium hydroxide (2 x 10mL), dried and evaporated to give the title compound.

### Method B

### 1-[(2-Piperidin-4-ylethyl)sulfonyl]piperidine hydrochloride

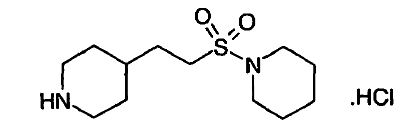

### Step 1: Preparation of 2-{1-[(benzyloxy)carbonyl]piperidin-4-yl}ethanesulfonic acid

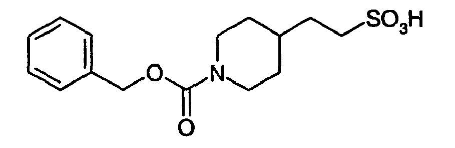

To 2-pyridin-4-ylethanesulfonic acid (20.0g, 107mmol) in water (200mL) was added concentrated ammonia solution (12mL) and 5% wt/wt rhodium on alumina (5g). The resulting mixture was hydrogenated under 5 atmospheres of hydrogen at 30°C. The mixture was filtered and sodium hydroxide pellets (15g) was added to the filtrate. The resulting mixture was concentrated to 120mL then cooled to 0°C. Benzyl chloroformate (20mL, 140mmol) was added dropwise with stirring. The resulting mixture was stirred for 1h with warming to room temperature. The mixture was washed with diethyl ether then the pH adjusted to 1 with concentrated hydrochloric acid. The mixture was extracted three times with a mixture of DCM and methanol (9:1). The combined extracts were dried and concentrated to give the sub-titled compound as a solid (6.5g); NMR: 0.9 (m, 2H), 1.5 (m, 3H), 1.6 (d, 2H), 2.2 (dd, 2H), 2.7 (m, 2H), 3.95 (d, 2H), 5.05 (s, 2H), 7.3 (m, 5H); LC-MS: 326 (M-H)⁻.

### Step 2: Preparation of 2-{1-[(benzyloxy)carbonyl]piperidin-4-yl}ethanesulfonyl chloride

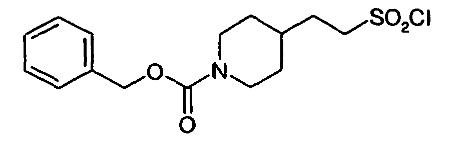

A mixture of 2-{1-[(benzyloxy)carbonyl]piperidin-4-yl}ethanesulfonic acid (6g) and thionyl chloride (50mL) was heated to reflux for 4h. The mixture was allowed to cool and the liquid was decanted and concentrated. The residue was azeotroped with toluene giving the sub-titled compound as a solid (5.9g); NMR (CDCl₃): 1.2 (m, 2H), 1.7 (m, 3H), 2.0 (m, 2H), 2.8 (dd, 2H), 3.7 (dd, 2H), 4.2 (d, 2H), 5.05 (s, 2H), 7.3 (m, 5H).

### Step 3: Preparation of benzyl 4-[2-(piperidin-1-ylsulfonyl)ethyl]piperidine-1-carboxylate

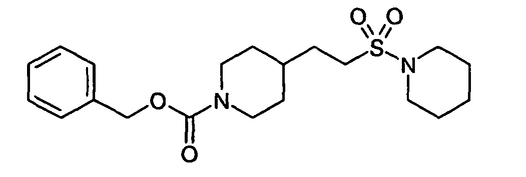

To a cooled (5°C) solution of 2-{ 1-[(benzyloxy)carbonyl]piperidin-4-yl}ethanesulfonyl chloride (5.5g) in DCM (100mL) was added piperidine (5.0mL) dropwise. The resulting mixture was stirred for 2h with warming to room temperature. The mixture was washed with 2M hydrochloric acid, dried (MgSO₄) and concentrated. The crude product was purified by silica gel chromatography to give the sub-titled compound (3.4g); NMR (CDCl₃): 1.15 (m, 2H), 1.6 (m, 9M, 1.75 (m, 2H), 2.8 (dd, 2H), 2.9 (dd, 3H), 3.25 (m, 4H), 4.2 (m, 2H), 5.1 (s, 2H), 7.35 (m, 5H); MS: 395.

### Step 4: Preparation of title compound

To a solution of benzyl 4-[2-(piperidin-1-ylsulfonyl)ethyl]piperidine-1-carboxylate (3.0g, 7.6mmol) in warm ethanol (30mL) was added concentrated hydrochloric acid (0.5mL) and 5% palladium on carbon (300mg). The resulting mixture was stirred under an atmosphere of hydrogen at room temperature for 24h. The mixture was filtered through Celite^{®}, rinsing with 10% aqueous ethanol. The combined filtrates were concentrated. The reside was triturated with ethyl acetate to give the title compound as a solid; MS: 261.

### Method C

### (R)-3-(3,5-Difluorophenyl)-3-[4-(methylsulfonyl)phenyl]propyl 4-methylbenzenesulfonate

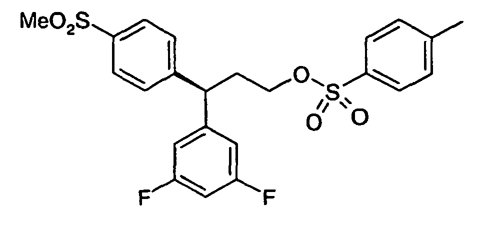

### Step 1: Preparation of (4S,5R)-1-[(R)-3-(4-methanesulfonyl-phenyl)-3-(3,5-di-fluorophenyl)-propionyl]-3,4-dimethyl-5-phenyl-imidazolidin-2-one

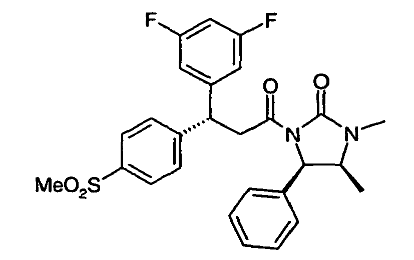

To a mixture of copper (I) iodide (5.01g, 26.3mmol) and THF (90mL) was added *N*,*N*,*N*',*N*'-tetramethylethylenediamine (4.2mL, 27.6mmol) and the resulting mixture was stirred at room temperature for 10min. then cooled to -78°C. 3,5-Difluorophenylmagnesium bromide (52mL, 0.5M in THF, 26.3mmol) was added and the resulting mixture stirred at -78°C for 30min. A solution of di-n-butylboron triflate (15.8mL, 1M in diethyl ether, 15.8mmol) and (*E*)-(4*S*, 5*R*)-1-(3-[4-methanesulfonylphenyl]acryloyl)-3,4-dimethyl-5-phenyl-imidazolidin-2-one (5.2 g, 13.1mmol) in THF (90mL) was added gradually and the resulting mixture was stirred whilst allowing to warm to room temperature for 18h. The reaction mixture was washed with saturated aqueous ammonium chloride then concentrated tetrasodium EDTA solution and evaporated to give a yellow solid. This was triturated with diethyl ether giving the sub-titled compound (4.04 g, 60%) as a white powder; NMR: 0.78 (d, 3H), 2.83 (s, 3H), 3.26 (s, 3H), 3.75 (dd, 1H), 4.05 (m, 2H), 4.80 (t, 1H), 5.35 (d, 1H), 7.10 (m, 3H), 7.20 (m, 2H), 7.35 (m, 3H), 7.73 (d, 2H), 7.93 (d, 2H); LC-MS: 513.

### Step 2: Preparation of (R)-3-(3,5-difluorophenyl)-3-[4-(methylsulfonyl)phenyl]propanol

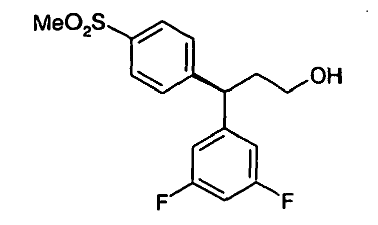

To a mixture of (4*S*,5*R*)-1-[(*R*)-3-(4-methanesulfonyl-phenyl)-3-(3,5-difluorophenyl)-propionyl]-3,4-dimethyl-5-phenyl-imidazolidin-2-one (57g, 111mmol) and THF (500mL) at 20°C was added lithium borohydride (2M in THF, 80mL, 160mmol) gradually. The resulting mixture was heated to reflux for 1h, cooled to 5°C and the reaction quenched by the gradual addition of 2M hydrochloric acid (200mL). The mixture was extracted with diethyl ether and the extracts dried and concentrated. The residue was triturated with ethyl acetate (200mL) and the resulting mixture filtered. The filtrate was concentrated and purified by silica column chromatography (eluting with ethyl acetate) to give the sub-titled compound as an oil (25.5g); NMR (CDCl₃): 1.65 (br s, 1H), 2.3 (m, 2H), 3.55 (m, 2H), 4.3 (t, 1H), 6.7 (m, 1H), 6.75 (m, 2H), 7.25 (d, 2H), 7.9 (d, 2H).

### Step 3: Preparation of title compound

To a solution of (*R*)-3-(3,5-difluorophenyl)-3-[4-(methylsulfonyl)phenyl]propanol (6.0g, 18.4mmol) in pyridine (50mL) was added tosyl chloride (3.9g, 20mmol). The resulting mixture was stirred at 20°C for 16h. The mixture was poured onto a mixture of ice and hydrochloric acid and the resulting mixture extracted with diethyl ether. The extracts were washed with 2M hydrochloric acid, water and aqueous potassium carbonate then dried (MgSO₄) and concentrated to give the title compound (6.6g); NMR (CDCl₃): 2.4 (m, 2H), 2.5 (s, 3H), 3.05 (s, 3H), 3.95 (t, 2H), 4.15 (d, 1H), 6.65 (m, 3H), 7.35 (m, 4H), 7.7 (d, 2H), 7.95 (d, 2H).

### Method D

### 3-Phenyl-3-(N-methanesulphonylpiperidin-4-yl)propionaldehyde

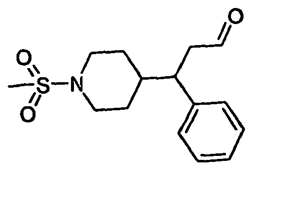

### Step 1: Preparation of 4-benzoyl-l-methanesulphonylpiperidine

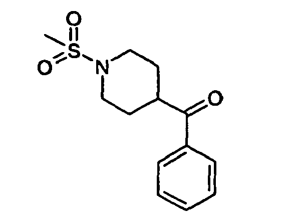

Methanesulphonyl chloride was added to a stirred slurry of 4-benzoylpiperidine hydrochloride (4.51g) and triethylamine (8.35ml) in dichloromethane (100m1) at 0°C. The reaction mixture was allowed to warm to room temperature and was stirred for 16 hours. The mixture was diluted with dichloromethane (50ml) and washed with ammonium chloride solution (2x25ml) and brine (25ml), dried and evaporated to dryness to give 4-benzoyl-1-methanesulphonylpiperidine as a white solid, yield 3.98g. NMR (CDCl₃): 1.93 (m, 4H), 2.81 (s, 3H), 2.98 (dt, 2H), 3.40 (m, 1H), 3.77 (m, 2H), 7.43 (t, 2H), 7.57 (t, 1H), 7.89 (d, 2H).

### Step 2: Preparation of ethyl 3-phenyl-3-(N-methanesulphonylpiperidin-4-yl)acrylate.

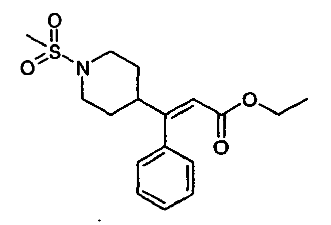

Lithium bis(trimethylsilyl)amide (16.3ml of a 1M solution in THF) was added dropwise to a solution of triethylphosphonoacetate (2.93ml) in THF at 0°C under an argon atmosphere and the mixture was stirred for 30 minutes. A slurry of 4-benzoyl-l-methanesulphonylpiperidine (3.96g) in THF (30ml) was added, the reaction mixture was allowed to warm to room temperature and stirring was continued for 24 hours. The reaction mixture was diluted with dichloromethane (80ml) and water (80ml). The organic layer was washed with water and the combined aqueous extracts were in turn extracted with dichloromethane (50ml). The combined dichloromethane extracts were washed with brine (25ml), dried and evaporated to dryness. The residue was chromatographed on a 90g Biotage column eluted with a solvent gradient (30-5-% ethyl acetate/isohexane to give a less polar fraction (1.62g) and a more polar fraction (0.53g). Both fractions (cis/trans isomers) were combined and used for the next step.

Less polar NMR (CDCl₃): 1.27 (t, 3H), 1.69 (m, 2H), 1.81 (d, 2H). 2.72 (s, 3H), 2.72 (t, 2H), 3.81 (d, 2H), 3.88 (m, 1H), 4.21 (q, 2H), 5.78 (s, 1H), 7.11 (m, 2H), 7.27 (m, 3H).

More polar NMR (CDCl₃): 1.01 (t,3H), 1.56 (m, 2H), 1.85 (d, 2H), 2.31 (m, 1H), 2.63 (t, 2H), 2.74 (s, 3H), 3.83 (d, 2H), 3.92 (q, 3H), 5.82 (s, 1H), 7.04 (d, 2H), 7.30 (m, 3H).

### Step 3: Preparation of ethyl 3-phenyl-3-(N-methanesulphonylpiperidin-4-yl)propionate

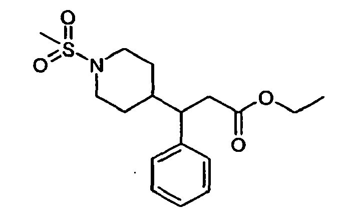

A solution of ethyl 3-phenyl-3-(N-methanesulphonylpiperidin-4-yl)acrylate (2.06g) in ethanol (30ml) was hydrogenated over 24 hours under a hydrogen filled balloon using 20% palladium hydroxide as catalyst. The reaction mixture was filtered through Celite^{®} and the filtrate evaporated to dryness. The product obtained was used for the next step without further purification. MS: 340.

### Step 4: 3-Phenyl-3-(N-methanesulphonylpiperidin-4-yl)propan-1-ol

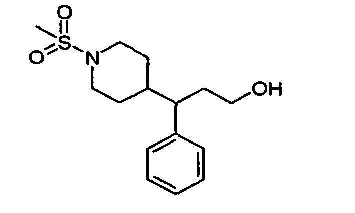

A solution of ethyl 3-phenyl-3-(N-methanesulphonylpiperidin-4-yl)propionate (2g) in THF (10ml) was added to a suspension of lithium aluminium hydride (232mg) in THF (20ml) at 0°C under argon over 30 minutes. The reaction mixture was allowed to warm to room temperature and stirred for 2 hours. Water (10ml) was added followed by magnesium sulphate (10g). The reaction mixture was filtered and the filtrate evaporated to dryness to give the product as a white foam, yield 1.57g. NMR (CDCl₃): 1.40 (m, 4H), 1.57 (m, 1H), 1.78 (m, 1H), 2.01 (m, 2H), 2.45 (m, 2H), 2.58 (t, 1H), 2.70 (m, 3H), 3.31 (m, 1H), 3.42 (m, 1H), 3.67 (d, 1H), 3.80 (d, 1H), 7.04 (d, 1H), 7.19 (t, 1H), 7.29 (q, 2H).

### Step 5: Preparation of the title compound

Dess-Martin periodinane (739mg) was added to a stirred solution of 3-phenyl-3-(N-methanesulphonylpiperidin-4-yl)propan-1-ol (454mg) in dichloromethane (8ml) and stirring was continued for 2 hours. The reaction mixture was diluted with dichloromethane (100ml) and washed with 2M sodium hydroxide (2x50ml), brine (50ml) and dried. The product obtained on removal of the solvent was used in subsequent steps without purification.

### Method E

### (R)-3-(3,5-Difluorophenyl)-3-(4-methanesulfonylphenyl)propionaldehdye

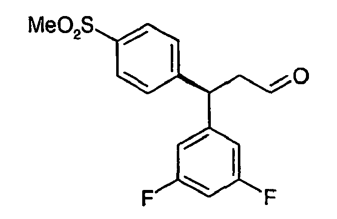

This was prepared from (4*S*, 5*R*)-1-[(*R*)-3-(4-methanesulfonyl-phenyl)-3-(3,5-difluorophenyl)-propionyl]-3,4-dimethyl-5-phenyl-imidazolidin-2-one using a method similar to that used to prepare (*S*)-3-phenyl-3-(4-methanesulfonyl-phenyl)propionaldehyde from (4*S*, 5*R*)-1-[(*S*)-3-(4-methanesulfonyl-phenyl)-3-phenyl-propionyl]-3,4-dimethyl-5-phenyl-imidazolidin-2-one (Method A); NMR (CDCl₃): 3.05 (s, 3H), 3.20 (d, 2H), 4.72 (t, 1H), 6.75 (m, 3H), 7.35 (d, 2H), 7.88 (d, 2H), 9.75 (s,1H).

### Method F

### (R)-3-(1-Methanesulphonylpiperidin-4-yl)-3-[3,5-difluorophenyl]propionaldehyde

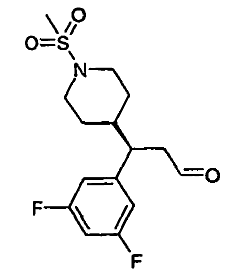

### Step 1: 3-[N-(benzyloxycarbonylpiperidin-4-yl)]propenoic acid

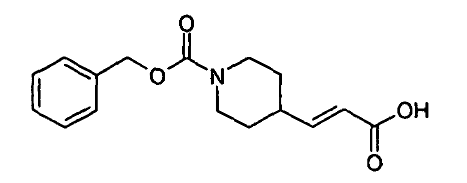

A mixture of N-benzyloxycarbonyl-4-formylpiperidine (10g), malonic acid (4.2), pyridine (4 ml) and piperidine (0.4 ml) was heated at 100°C for 2 hours. The reaction mixture was allowed to cool and was diluted with ethyl acetate (100 ml). The solution was washed with 2M HCl (2x100 ml), dried and evaporated to dryness. The residue was triturated with isohexane to give the title compound, yield 13.5g; NMR (d6-DMSO): 1.2 (m, 2H) 1.7 (m, 2H) 2.35 (m, 1H) 2.85 (m, 2H) 4 (d, 2H) 5.05 (s, 2H) 5.75 (d, 1H) 6.75 (m, 1H) 7.35 (m, 5H) 12.25 (br, 1H).

### Step 2: N-(benzyloxycarbonylpiperidin-4-yl)propenoic acid isopropyl ester

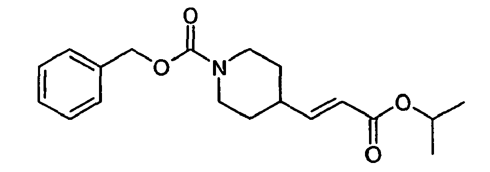

A solution of N-(benzyloxycarbonylpiperidin-4-yl)propenoic acid (52g) in isopropanol (500 ml) containing concentrated sulphuric acid (20 ml) was heated under reflux for 32 hours. The solvent was evaporated and the residue was dissolved in ethyl acetate (250 ml). The ethyl acetate solution was washed with water (2x250 ml) and saturated aqueous sodium bicarbonate (2x25 ml) and dried. The residue obtained on evaporation of the solvent was chromatographed on a Bond Elut cartridge eluted with a solvent gradient (isohexane-25% ethyl acetate/isohexane) to give the title compound, yield 54g.

### Step 3: Preparation of (R)-3-(N-benzyloxycarbonylpiperidin-4-yl)-3-(3,5-difluorophenyl)propanoic acid isopropyl ester

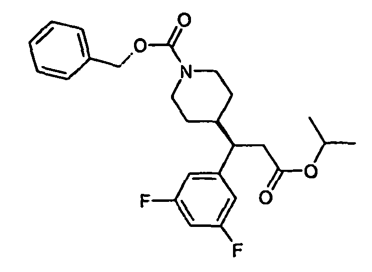

Dioxane (100 ml) was charged to a 500 ml three necked flask and purged with argon for 10 minutes. Acetylacetonatobis[ethylene]rhodium (1) (620 mg) and R-BINAP were added and the mixture was stirred for 10 minutes. 3,5-Difluorophenylboronic acid (19g) was added and the mixture was purged with argon for 10 minutes. N-(benzyloxycarbonylpiperidin-4-yl)propenoic acid isopropyl ester (8 g) and ethanediol (20 ml) in dioxane (100 ml) were added and the mixture was purged with argon for 10 minutes. The mixture was heated at 100°C for 18 hours, allowed to cool and was passed through activated alumina (200g) washed through with ethyl acetate (3x100 ml). The combined washings were evaporated to dryness and the residue obtained was dissolved in ethyl acetate (100 ml) and washed successively with saturated aqueous sodium bicarbonate (2x100 ml) and 2M HCI (2x100 ml), dried and evaporated to dryness. The product obtained (12g) was shown to be 40% of the required material by NMR and was used without further purification in the subsequent reactions.

### Step 4: Preparation of (R)-3-(piperidin-4-yl)-3-(3,5-difluorophenyl)propanoic acid isopropyl ester

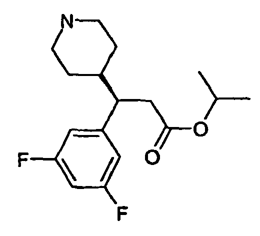

A solution of (*R*)-3-(N-benzyloxycarbonylpiperidin-4-yl)-3-(3,5-difluorophenyl)propanoic acid isopropyl ester (12g) in ethanol (300 ml) containing 20% palladium hydroxide on charcoal (2g) was hydrogenated under a hydrogen filled balloon. The catalyst was filtered and the filtrate was evaporated to dryness to give the title compound (10g) which was used without further purification.

### Step 5: Preparation of (R)-3-(N-methanesulphonylpiperidin-4-yl)-3-(3,5-difluorophenyl)-propanoic acid isopropyl ester

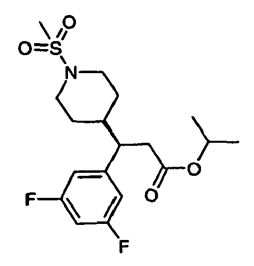

Methanesulphonyl chloride (3.7g) was added to a solution of (*R*)-3-(piperidin-4-yl)-3-(3,5-difluorophenyl)propanoic acid isopropyl ester (10g) and triethylamine (3.89g) in dichloromethane (100 ml) at 0°C. The reaction mixture was allowed to warm to room temperature and was washed with 2M HCl (2x50 ml) and saturated aqueous sodium bicarbonate (2x50 ml), dried and evaporated to dryness to give the title compound (10g) which was used without further purification.

### Step 6: Preparation of (R)-3-(N-methanesulphonylpiperidin-4-yl)-3-(3,5-difluorophenyl)propanol

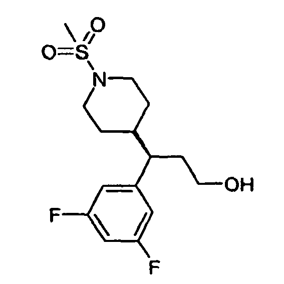

Lithium aluminium hydride (25 ml of a 1M solution in THF) was added dropwise over 15 minutes to a solution of (*R*)-3-(N-methanesulphonylpiperidin-4-yl)-3-(3,5-difluorophenyl)propanoic acid isopropyl ester (10g) in THF (150 ml) at -10°C. The reaction mixture was stirred at -10°C for 30 minutes, 2M NaOH (25 ml) was added, the mixture was filtered and the filtrate evaporated to dryness. The residue obtained was dissolved in ethyl acetate and washed with 2M HCl (2x100 ml) and dried. The residue obtained on removal of the solvent was chromatographed on a Bond Elut column eluting with a solvent gradient (80% ethyl acetate/isohexane-ethyl acetate) to give the title compound, yield 2.2 g; NMR (d6-DMSO): 0.95-1.2 (m, 2H) 1.3 (m, 1H) 1.6 (m.2H) 1.9 (m, 2H) 2.6 (m, 2H) 2.8 (s, 3H) 3.1 (m, 1H) 3.2 (m, 1H) 3.4 (m, 1H) 3.5 (m, 1H) 6.8-7.0 (m, 3H).

### Step 7: Preparation of title compound

Dess-Martin periodinane (1g) was added to a solution of (R)-3-(N-methanesulphonylpiperidin-4-yl)-3-(3,5-difluorophenyl)propanol (0.8g) in dichloromethane (40 ml) and the mixture was stirred for 1.5 hours. The reaction mixture was washed with 2M NaOH (2x20 ml) and dried. The solution of the title compound in dichloromethane was used in subsequent reactions.

### Method G

### (R)-3-(N-Methanesulphonylpiperidin-4-yl)-3-phenylpropanol

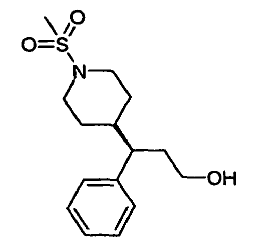

### Step 1: Preparation of 3-(N-methanesulphonylpiperidin-4-yl)propenoic acid chloride.

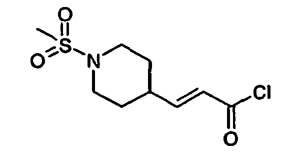

1-Chloro-N,N,2-trimethylpropenylamine (1.06 ml) was added dropwise over 10 minutes to a suspension of 3-(N-methanesulphonylpiperidin-4-yl)propenoic acid (1.86g, prepared from N-methanesulphonylpiperidine-4-carboxaldehyde [CAS 241134-35-0] according to step 1 of Method F) in THF (20 ml) under an atmosphere of argon and the mixture was stirred for 2 hours and used directly in step 2.

### Step 2: Preparation of 1-[3-(N-methanesulphonylpiperidin-4-yl)propenyl]-(4S,5R)-3,4-dimethyl-4-phenyl-imidazolidin-2-one

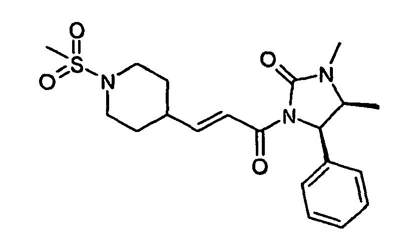

Lithium bis(trimethylsilyl)amide (8 ml of a 1M solution in THF) was added dropwise to a suspension of (4*R*,5*S*)-1,5-dimethyl-4-phenyl-2-imidazolidinone (1.52g) in THF (20 ml) under argon at -10°C. the reaction mixture was stirred at -10°C for 10 minutes, allowed to warm to 0°C and maintained at this temperature for 10 minutes then cooled again to -10°C. The solution of the acid chloride prepared in Step 1 was added dropwise and the reaction mixture was allowed to warm to room temperature and washed with water (100 ml). The aqueous extract was extracted with ethyl acetate (3X50 ml) and the ethyl acetate extracts were dried and the residue passed through a 90g Biotage column eluting with a solvent gradient (50% ethyl acetate/isohexane-70% ethyl acetate/isohexane). Yield 1.89g; NMR (CDCl₃): 0.8 (d, 3H) 1.5-1.6 (m, 3H) 1.9 (m, 2H) 2.3 (m, 1H), 2.7 (m, 2H) 2.75 (s, 3H) 2.8 (s, 3H) 3.75 (m, 2H) 3.9 (m, 1H) 5.3 (d, 1H) 6.85 (d-d, 1H) 7.1 (d, 1H) 7.2-7.35 (m, 3H) 7.45 (d, 1H).

### Step 3: Preparation of (R)-1-[3-phenyl-3-(methanesulphonylpiperidin-4-yl)propionyl]-(4S,5R)-3,4-dimethyl-5-phenyl-imidazolidin-2-one

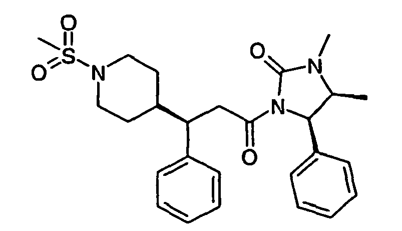

A mixture of copper(I) iodide (1.78 g) and *N,N,N'N'*-tetramethylethylenediamine (1.41 ml) in THF (50 ml) was stirred under argon for 1 hour then cooled to -78°C and phenylmagnesium bromide (5.4 ml of a 1M solution in THF) was added and the mixture was stirred at -78°C for 30 minutes. A solution of 1-[3-(N-methanesulphonylpiperidin-4-yl)propenyl]-(4*S*, 5*R*)-3,4-dimethyl-5-phenyl-imidazolidin-2-one (1.89g) and dibutylboron triflate (4.67 ml of a 1M solution in diethylether in THF (50 ml) was added over 10 minutes and the reaction mixture was stirred at -78°C for 1 hour then allowed to warm to room temperature. The reaction mixture was concentrated and filtered through a pad of silica (50g) washed with ethyl acetate (2x50 ml) and the ethyl acetate washings were washed with 2M HCl (2x150 ml) and dried. The residue obtained on removal of the solvent was passed through a 90g Biotage column eluting with a solvent gradient (50% ethyl acetate/isohexane-70% ethyl acetate/isohexane) to give the product as a yellow solid, yield 1.34g; NMR (CDCl₃): 0.7 (d, 3H) 1.2 (m, 1H) 1.35 (m, 1H) 1.5 (m, 1H) 1.9 (m, 1H) 2.45 (m, 1H) 2.55 (m, 1H) 2.7 (s, 3H) 2.8 (s, 3H) 3.1 (m, 1H) 3.2 (d-d, 1H) 3.4 (m, 1H) 3.65 (m, 1H) 3.75-3.9 (m, 3H) 5.2 (d, 1H) 6.7 (d, 2H) 7.05-7.25 (m, 8H). MS: 484.

### Step 4: Preparation of the title compound

A solution of (*R*)-1-[3-phenyl-3-(methanesulphonylpiperidin-4-yl)propionyl]-(4*S*,5*R*)-3,4-dimethyl-5-phenyl-imidazolidin-2-one (1.34g) in THF (14 ml) was added to a solution of lithium aluminium hydride (2.77 ml of a 1M solution in THF) in THF (10 ml) at 0°C and the mixture was allowed to warm to room temperature over 1 hour. Water (5 ml) was added cautiously, then THF (15 ml) and solid magnesium sulphate. The reaction mixture was filtered and the filtrate was passed through a 40 g Biotage column eluted with a solvent gradient (50% ethyl acetate/isohexane-70% ethyl acetate/isohexane) to give the title compound as a white solid, yield 338 mg; NMR (CDCl₃): 1.15-1.25 (m, 2H) 1.3-1.5 (m, 2H) 1.6 (m, 1H) 1.75 (m, 1H) 1.95-2.10 (m, 2H) 2.5 (m, 2H) 2.6 (m, 1H) 2.7 (s, 3H) 3.3-3.4 (m, 2H) 3.45 (m, 1H) 3.7 (m, 1H) 3.85 (m, 1H) 7.05 (m, 2H) 7.15-7.35 (m, 3H).

### Method H

### Preparation of benzyl 4-{[2-(1-{(3R)-3-(3,5-difluorophenyl)-3-[4-(methylsulfonyl)phenyl]propyl}piperidin-4-yl)ethyl]sulfonyl}piperidine-1-carboxylate

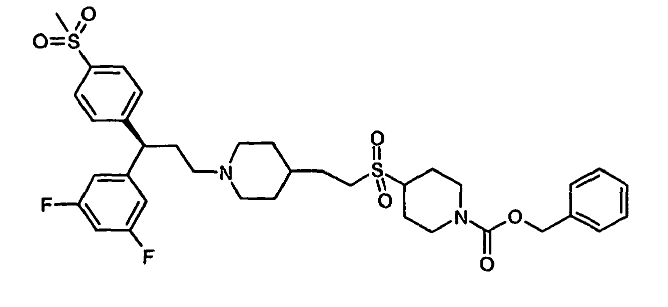

### Step 1 Preparation of benzyl 4-(acetylthio)piperidine-1-carboxylate

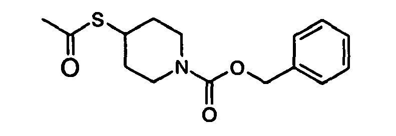

Methanesulphonyl chloride (3.47 ml) was added to a solution of benzyl 4-hydroxypiperidine-1-carboxylate (9.4 g) in dichloromethane (140 ml) containing triethylamine (11.2 ml) at 0 °C under argon and the mixture was allowed to warm to room temperature and was stirred for 48 hours, diluted with dichloromethane (200 ml) and washed with ammonium chloride solution (2x200 ml) and dried. The solvent was evaporated to dryness and the residue obtained was dissolved in dichloromethane (200 ml) and potassium thioacetate (9.14 g) was added. The mixture was heated at 90 °C for 3 hours, allowed to cool and evaporated to dryness. The residue was dissolved in ethyl acetate (200 ml) and washed with water (300 ml) brine (200 ml) and dried. The residue obtained on removal of the solvent was purified on a 350 g Biotage silica column eluting with a solvent gradient of isohexane: 20% ethyl acetate/isohexane to give the product, yield 8.07 g, LC-MS M⁺-Acetyl 250. NMR (CDCl₃): 1.58 (2H, m), 1.92 (2H, m), 2.32 (3H, s), 3.14 (2H, m), 3.62 (1H, m), 3.94 (2H, d), 5.12 (3H, s), 7.32 (5H, m).

### Step 2 Preparation of benzyl 4-mercaptopiperidine-1-carboxylate

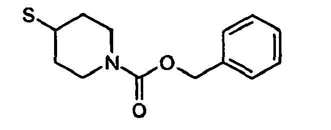

Sodium borohydride (2.08 g) was added in portions to a solution of benzyl 4-(acetylthio)piperidine-1-carboxylate (8.07 g) in methanol (135 ml) at 0 °C. The reaction mixture was allowed to warm to room temperature and was stirred for 1 hour. Additional sodium borohydride (1.04 g) was added and stirring was continued for 30 minutes. The reaction mixture was evaporated to small volume and the residue partitioned between dichloromethane (50 ml) and 10% citric acid solution (50 ml). The dichloromethane layer was collected and washed with 10% citric acid solution (25 ml) and brine (25 ml) and dried. The residue obtained on removal of the solvent was purified by chromatography on silica eluting with a mixture of ethyl acetate and isohexane (1:5) to give the product as a yellow oil, yield 5.09g, LC-MS MH⁺ 252. NMR (CDCl₃): 1.56 (3H, m), 1.98 (2H, d), 2.96 (3H, m), 4.04 (2H, d), 3.94 (2H, d), 5.12 (3H, s), 7.34 (5H, m).

### Step 3 Preparation of tert-butyl 4-[2-({1-[(benzyloxy)carbonyl]piperidin-4-yl}sulfonyl)ethyl]piperidine-1-carboxylate.

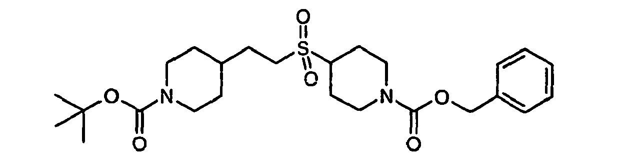

A solution of benzyl 4-mercaptopiperidine-1-carboxylate (2.51 g) in DMF (10 ml) was added to a suspension of sodium hydride (440 mg of a 50% dispersion in mineral oil) in DMF (10 ml) at 0 °C under an atmosphere of argon and was allowed to warm to room temperature and was stirred for 30 minutes. A solution of *tert-*butyl 4*-*(2-{[(4-methylphenyl)sulfonyl]oxy}-ethyl)piperidine-1-carboxylate (3.82 g) in DMF (10 ml) was added and the mixture was stirred for 16 hours and then evaporated to dryness. The residue obtained on removal of the solvent was dissolved in dichloromethane ((50 ml) and cooled to 0 °C and solid meta-chloroperbenzoic acid was added in portions. The mixture was allowed to warm to room temperature and was stirred for 48 hours. Dichloromethane (200 ml) was added and the mixture was washed with 2M NaOH (2x100 ml) and brine (100 ml) and dried. The residue obtained on removal of the solvent was purified by chromatography on silica eluting with a solvent gradient of 50% ethyl acetate/isohexane to 70% ethyl acetate/isohexane. The product was obtained as a colourless oil, yield 2.02 g, LC-MS MH⁺ 395.

### Step 4 Preparation of benzyl 4-[(2-piperidin-4-ylethyl)sulfonyl]piperidine-1-carboxylate.

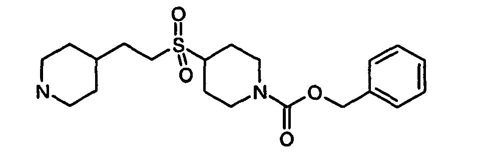

A solution of tert-butyl 4-[2-({1-[(benzyloxy)carbonyl]piperidin-4-yl}sulfonyl)ethyl]piperidine-1-carboxylate (2.02 g) in 4M HCl in dioxane (41 ml) was stirred for 1 hour and evaporated to dryness. The residue was dissolved in 2M NaOH (50 ml) and extracted with dichloromethane (2x50 ml). The organics were collected and dried and on evaporation to dryness gave the title compound as an off white solid, yield 1.41g, LC-MS MH⁺ 395. NMR (CDCl₃): 1.31 (3H, m), 1.57-1.96 (6H, m), 2.09 (2H, d), 2.68 (2H, t), 2.78-3.01 (6H, m), 3.22 (1H, d), 4.36 (2H, (br m), 5.11 (2H, s), 7.33 (5H, m).

### Step 5 Preparation of title compound

MP-triacetoxyborohydride (3.58 g) was added to a mixture of (3*R*)-3-(3,5-difluorophenyl)-3-[4-(methylsulfonyl)phenyl]propanal (1.3 g) (Method A) and benzyl 4-[(2-piperidin-4-ylethyl)sulfonyl]piperidine-1-carboxylate (1.41 g) (Step 4) in dichloromethane (36 ml) and the mixture was stirred for 18 hours at room temperature. The resin was filtered and washed with 10% methanol in dichloromethane (20 ml) and the combined organics were evaporated to dryness. The residue was purified on the ISCO companion (silica, 120 g) using a solvent gradient of ethyl acetate : 20% methanol/ethyl acetate to give the title compound as a white foam, yield 1.52g, LC-MS MH⁺ 703. NMR (CDCl₃) 1.30 (3H, m), 1.62-3.34 (15H, m), 2.78-3.00 (9H, m), 3.02 (3H, s), 4.08 (1H, m), 4.36 (2H, m), 5.14 (2H, s), 6.62-6.78 (3H, m), 7.34 (5H, m), 7.40 (2H, d), 7.84 (2H, d).

Using the method described in step 5 with (3*R*)-3-(3,5-difluorophenyl)-3-[1-(methylsulfonyl)piperidin-4-yl]propanal as starting material (Method G, followed by Dess-Martin oxidation as described in Method F, step 7) there is obtained benzyl 4-{[2-(1-{(3R)-3-(3,5-difluorophenyl)-3-[1-(methylsulfonyl)piperidin-4-yl]propyl}piperidin-4-yl)ethyl]sulfonyl}piperidine-1-carboxylate, LC-MS MH⁺ 710. NMR (CDCl₃) 1.16-2.24 (22H, m), 2.38 (1H, t), 2.50 (1H, m), 2.61 (1H, m), 2.74 (3H, s), 2.74-3.04 (7H, m), 3.72 (1H, m), 3.84 (1H, m), 4.36 (2H, m), 5.16 (2H, s), 6.62 (3H, m), 7.36 (5H, m).

### Method I

### Preparation of 2-(1-{(3R)-3-(3,5-difluorophenyl)-3-[4-(methylsulfonyl)phenyl]propyl}-4-piperidinyl)ethanesulfonyl chloride.

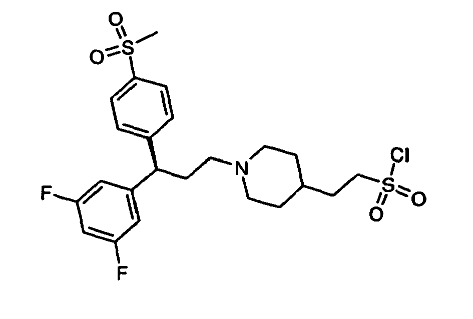

### Step 1 Preparation of tert-butyl 4-[2-(acetylsulfanyl)ethyl]-1-piperidinecarboxylate

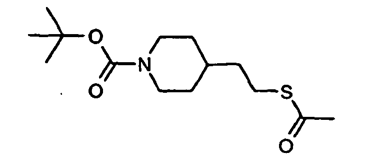

To a solution of tert-butyl 4-(2-{ [(4-methylphenyl)sulfonyl]oxy}ethyl)-1-piperidinecarboxylate(15.34g, 40mmol) in dry THF (100ml), under Argon, was added potassium thioacetate (4.57g, 40mmol) and the mixture heated to 50°C for 2 hours, with the formation of a white precipitate. MTBE (100ml) was added and the suspension filtered and the filter cake washed with further MTBE. The combined filtrates were purified by chromatography on silica (50g, 0-20% EtOAc/ iso-hexane eluent) to give the title compound as a yellow mobile oil, 8.91g, 78% yield. NMR (CDCl₃) 1.04(m, 2H), 1.38(s, 9H), 1.45(m, 2H), 1.52 (m,1H) 1.62(d, 2H), 2.25(s, 3H), 2.62(t, 2H), 2.82(m, 2H), 4.01(m, 2H); MS: 188 (M+H-BOC)⁺.

### Step 2 Preparation of 4-[2-(acetylsulfanyl)ethyl]piperidinium chloride

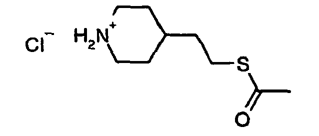

tert-Butyl 4-[2-(acetylsulfanyl)ethyl]-1-piperidinecarboxylate(8.91g, 31.00mmol) was dissolved in 10% HCl / Methanol (25ml) and the solution heated to 60°C for 1 hour. The solvent was evaporated and residue azeotroped with toluene (100ml) to give the title compound as a yellow powder, 6.10g, 88% yield; NMR (400Mz, CDCl₃), 8/ppm: 1.81 (m, 4H), 2.49 (s, 3H), 2.82 (q, 4H), 3.43 (d, 4H), 4.30 (br s, 1H), 9.18 (br s, 1H), 9.41 (br s, 1H) MS: 188 (MH)⁺.

### Step 2 Preparation of S-[2-(1-{(3R)-3-(3,5-difluorophenyl)-3-[4-(methylsulfonyl)phenyl]-propyl }-4-piperidinyl)ethyl] ethanethioate

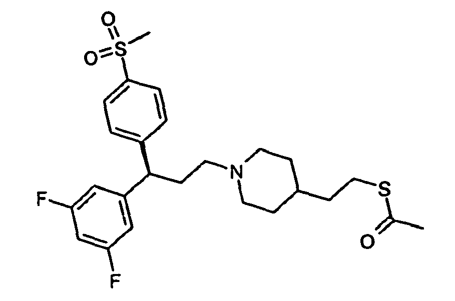

To a solution of 4-[2-(acetylsulfanyl)ethyl]piperidinium chloride (3.39g, 15.2mmol) and (3*R*)-3-(3,5-difluorophenyl)-3-[4-(methylsulfonyl)phenyl]propanal(4.90g, 15.2mmol) in dichloromethane (100ml) was added NaBH(OAc)₃ (3.22g, 15.2mmol) and the suspension stirred at room temperature for 30 minutes. Saturated Sodium Bicarbonate (5ml) was added and the mixture stirred at room temperature overnight. Acetic Anhydride (10ml) and Triethylamine (10ml) were added, stirring for 30minutes then the organic layer separated and washed with saturated sodium bicarbonate(3x100ml), dried and purified by chromatography on silica (90g, 0-20% EtOH / EtOAc eluent) to give the title compound as a yellow oil, 3.66g, 49% yield; NMR (400Mz, CDCl₃), δ/ppm: 1.26(m, 2H), 1.33(m, 2H), 1.52 (q, 2H) 1.70 (d, 2H), 1.96(m,2H), 2.28 (m, 3H), 2.32 (s, 3H), 2.88 (t, 2H) 2.91 (m, 2H), 3.03 (s, 3H), 3.21 (m, 2H), 4.09 (t, 1H), 6.59 (t,1H), 6.67 (d, 2H), 7.34 (d, 2H), 7.80 (d, 2H); MS: 496 (MH)⁺.

### Step 3 Preparation of the title compound

A solution of *S*-[2-(1-{(3*R*)-3-(3,5-difluorophenyl)-3-[4-(methylsulfonyl)phenyl]-propyl }-4-piperidinyl)ethyl] ethanethioate(539mg, 1.09mmol) in AcOH (5ml) / water (0.5ml) was cooled to 0 °C and chlorine was bubbled through the solution for 5 minutes followed by argon. The solvents were evaporated and the residue azeotroped with toluene (3x50ml), redissolved in dichloromethane(100ml) / isohexane (100ml) and evaporated to give the title compound as a white foam, 553mg, 97%yield; MS: 520, 522 (MH+) (Cl isotopes).

### Example 7

The ability of compounds to inhibit the binding of MIP-la was assessed by an *in vitro* radioligand binding assay. Membranes were prepared from Chinese hamster ovary cells which expressed the recombinant human CCR5 receptor. These membranes were incubated with 0.1nM iodinated MIP-1α, scintillation proximity beads and various concentrations of the compounds of the invention in 96-well plates. The amount of iodinated MIP-1α bound to the receptor was determined by scintillation counting. Competition curves were obtained for compounds and the concentration of compound which displaced 50% of bound iodinated MIP-1α was calculated (IC₅₀). Certain compounds of formula (I) have an IC₅₀ of less than 50µM.

Results from this test for certain compounds of the invention are presented in Table IV. In Table IV the results are presented as Pic50 values. A Pic50 value is the negative log (to base 10) of the IC₅₀ result, so an IC50 of 1µM (that is 1 x 10⁻⁶M) gives a Pic50 of 6. If a compound was tested more than once then the data below is an average of the probative tests results.

**TABLE IV**

| Table Number | Compound number | Pic50 |
|---|---|---|
| I | 2 | 7.4 |
| I | 3 | 8.4 |
| III | 1 | 8.5 |
| III | 13 | 8.0 |
| III | 15 | 8.3 |
| III | 19 | 8.6 |
| III | 53 | 8.9 |
| III | 54 | 9.2 |

### Scheme 1

To prepare compounds of the invention, for example wherein R¹ is aryl or C-linked heterocyclyl. 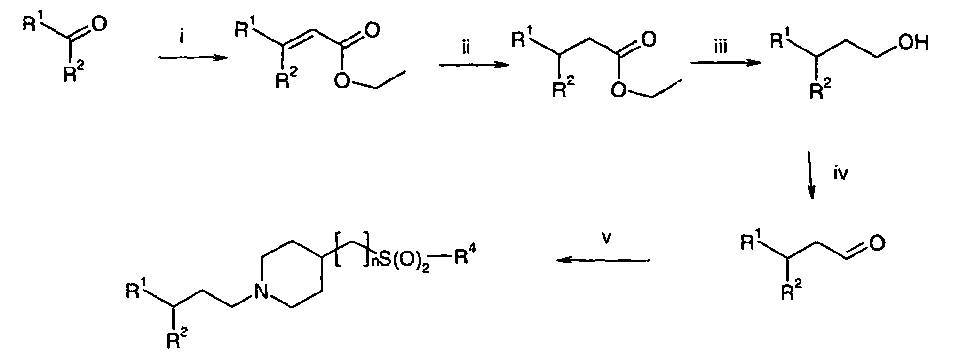
i Wittig reaction (eg LHDMS, triethylphosphonoacetate)
ii Catalytic hydrogenation (eg H₂, 10% Pd/C)
iii Reduction (eg LAH)
iv Oxidation (eg Dess-Martin oxidation)
v reductive amination with 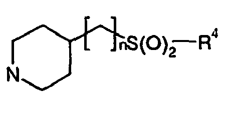 (eg using sodium triacetoxyborohydride)

### Scheme 2

To prepare compounds of the invention, for example wherein R¹ is aryl or C-linked heterocyclyl. 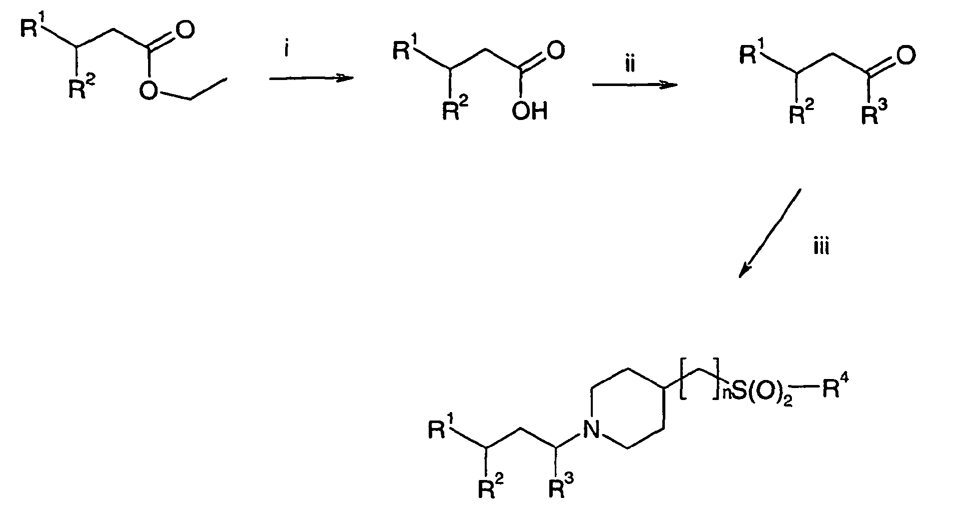
i Base hydrolysis (eg LiOH, MeOH/H₂O)
ii MeMgCl, R³MgBr, Et₂O
iii reductive amination 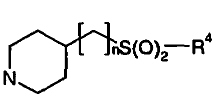 in presence of titanium tetra-isopropoxide (eg using sodium triacetoxyborohydride)

### Scheme 3

To prepare compounds of the invention, for example wherein R¹ is aryl, heteroaryl, heterocyclyl or NR¹³C(O)R¹⁴. 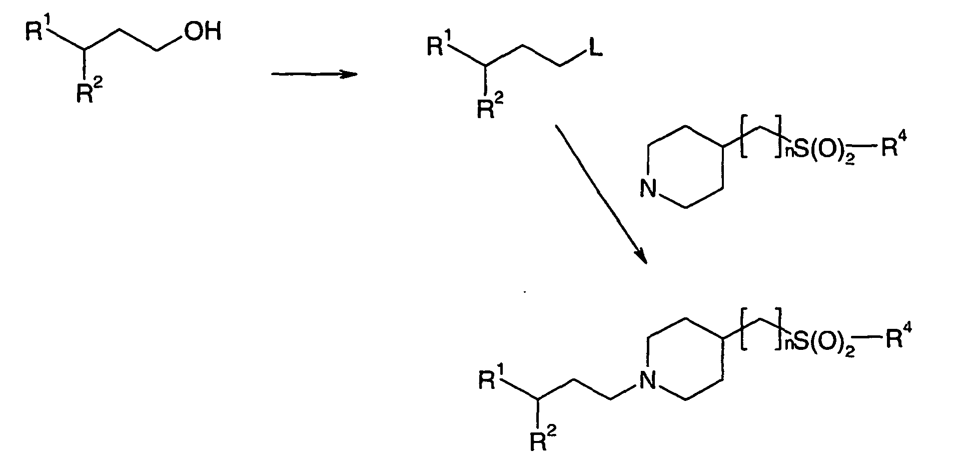 in which L is an activated group, such as halogen, mesylate, tosylate or triflate.

### Scheme 4

To prepare compounds of the invention, for example wherein R¹ is aryl, heteroaryl or heterocyclyl. 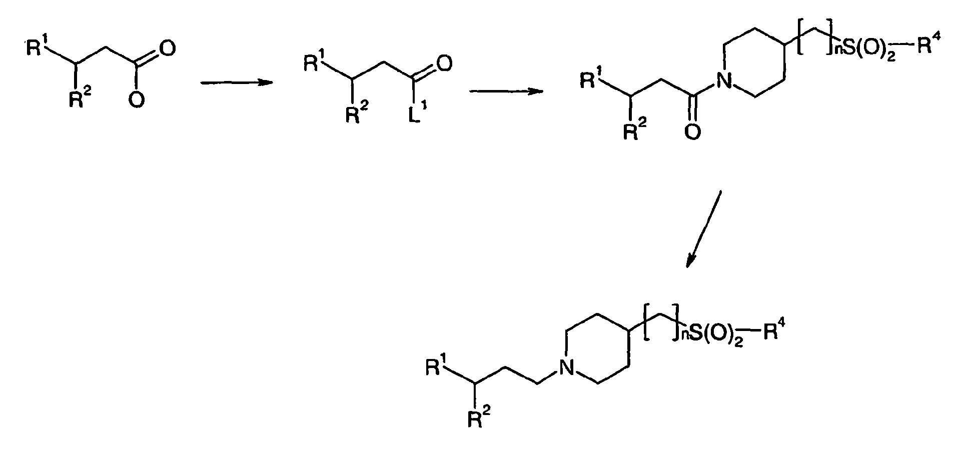 in which L¹ is a halogen, an activated ester or a complex formed with a carbodiimide.

### Scheme 5

To prepare compounds of the invention, for example wherein R¹ is NR¹³C(O)R¹⁴. 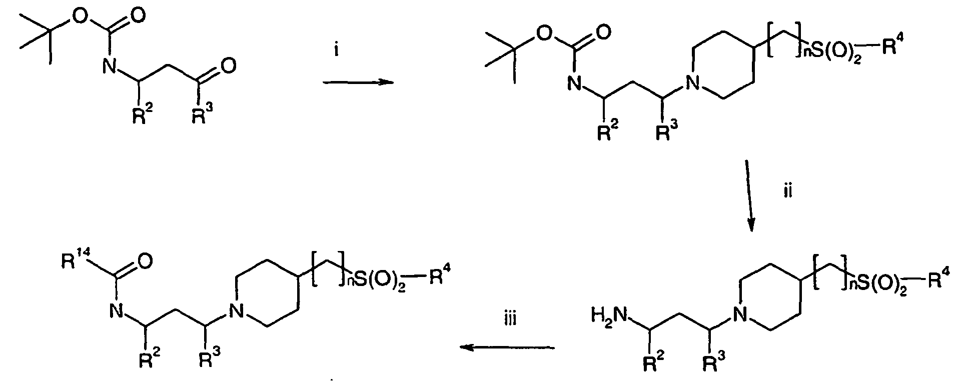
i reductive amination (if R³ is H can use sodium triacetoxyborohydride; if R³ is alkyl can use titanium tetra-isopropoxide and sodium triacetoxyborohydride)
ii Deprotection (eg TFA)
iii amide bond formation (eg acid chloride, active ester or carbodiimide mediated)

### Scheme 6

To prepare compounds of the invention, for example wherein R¹ is piperazine. 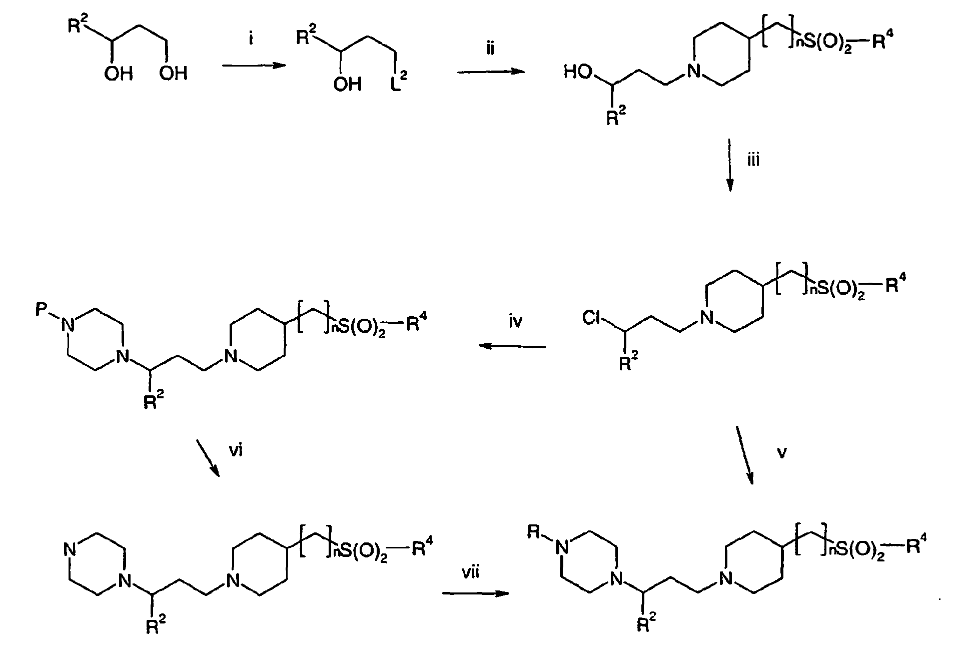
i Conversion of an OH to a leaving group (eg tosyl chloride (L² is Tosylate) or mesyl chloride (L² is Mesylate))
ii displacement reaction with 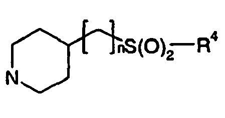 (eg in presence of triethylamine)
iii Mesyl chloride, DCM 0°C
iv Displacement reaction with mono-protected piperazine (P is a protecting group)
v Displacement reaction with R substituted piperazine
vi Deprotection (TFA for Boc, hydrogenation for Cbz)
vii Depending on R, acylation, sulphonylation, alkylation, reductive amination

### Scheme 7

To prepare compounds of the invention, for example wherein R¹ is aryl or piperidine. 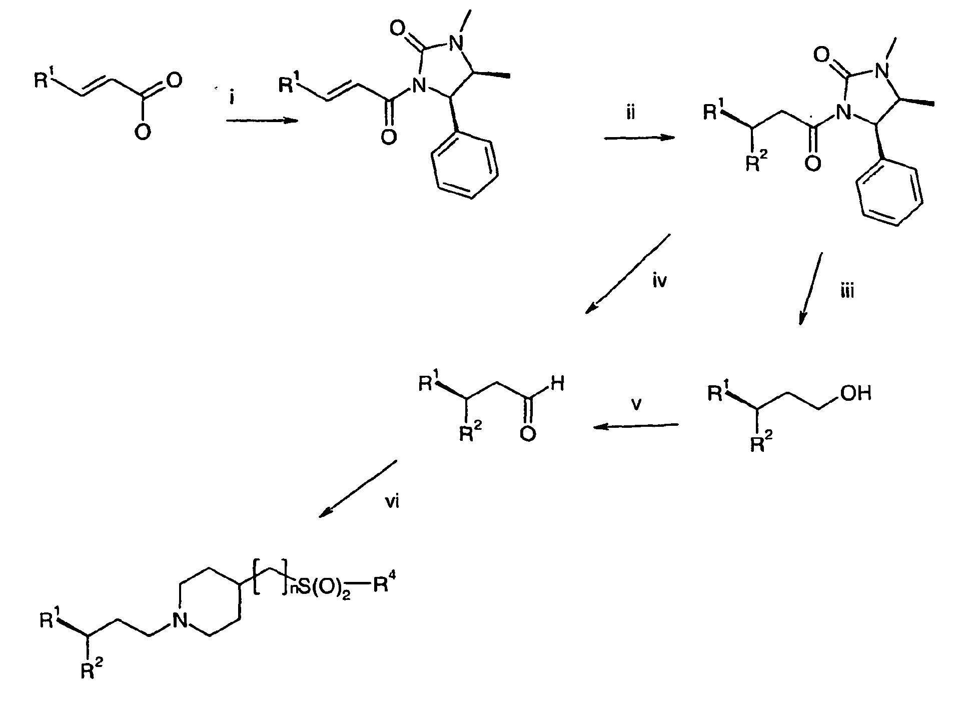
i activation of acid group and coupling with chiral auxiliary (eg SOCl₂, 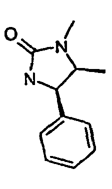 )
ii 1,4-addition of organocuprate (eg R²MgBr, Cu(I)I, TMEDA, di-butylboron triflate)
iii reduction (eg lithium aluminium hydride)
iv Dibal
v Oxidation (eg Dess-Martin reagent)
vi reductive amination (eg with sodium triacetoxyborohydride)

## Claims

1. A compound of formula (I): 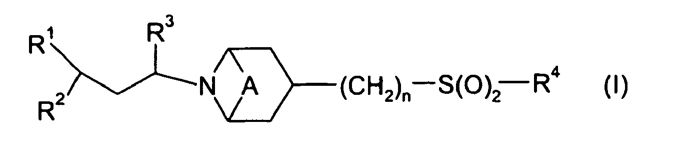 wherein:
A is absent or is (CH₂)₂;
R¹ is C(O)NR¹⁰R¹¹, C(O)₂R¹², NR¹³C(O)R¹⁴, NR¹⁵C(O)NR¹⁶R¹⁷, NR¹⁸C(O)₂R¹⁹, heterocyclyl, aryl or heteroaryl;
R¹⁰, R¹³, R¹⁵, R¹⁶ and R¹⁸ are hydrogen or C₁₋₆ alkyl;
R¹¹, R¹², R¹⁴, R¹⁷ and R¹⁹ are C₁₋₈ alkyl (optionally substituted by halo, hydroxy, C₁₋₆ alkoxy, C₁₋₆ haloalkoxy, C₃₋₆ cycloalkyl (optionally substituted by halo), C₅₋₆ cycloalkenyl, S(C₁₋₄ alkyl), S(O)(C₁₋₄ alkyl), S(O)₂(C₁₋₄ alkyl), heteroaryl, aryl, heteroaryloxy or aryloxy), aryl, heteroaryl, C₃₋₇ cycloalkyl (optionally substituted by halo or C₁₋₄ alkyl), C₄₋₇ cycloalkyl fused to a phenyl ring, C_{5-7.} cycloalkenyl, or, heterocyclyl (itself optionally substituted by oxo, C(O)(C₁₋₆ alkyl), S(O)ₖ(C₁₋₆ alkyl), halo or C₁₋₄ alkyl); or R¹¹, R¹², R¹⁴ and R¹⁷ can also be hydrogen;
or R¹⁰ and R¹¹, and/or R¹⁶ and R¹⁷ may join to form a 4-, 5- or 6-membered ring which optionally includes a nitrogen, oxygen or sulphur atom, said ring being optionally substituted by C₁₋₆ alkyl, S(O)₁(C₁₋₆ alkyl) or C(O)(C₁₋₆ alkyl);
R² is phenyl, heteroaryl or C₃₋₇ cycloalkyl;
R³ is H or C₁₋₄ alkyl;
R⁴ is heterocyclyl;
n is 1, 2 or 3;
aryl, phenyl and heteroaryl moieties are independently optionally substituted by one or more of halo, cyano, nitro, hydroxy, OC(O)NR²⁰R²¹, NR²²R²³, NR²⁴C(O)R²⁵, NR²⁶C(O)NR²⁷R²⁸, S(O)₂NR²⁹R³⁰, NR³¹S(O)₂R³², C(O)NR³³R³⁴, CO₂R³⁶, NR³⁷CO₂R³⁸, S(O)_{q}R³⁹, OS(O)₂R⁴⁹, C₁₋₆ alkyl (optionally mono-substituted by S(O)₂R⁵⁰ or C(O)NR⁵¹R⁵²), C₂₋₆ alkenyl, C₂₋₆ alkynyl, C₃₋₁₀ cycloalkyl, C₁₋₆ haloalkyl, C₁₋₆ alkoxy(C₁₋₆)alkyl, C₁₋₆ alkoxy, C₁₋₆ haloalkoxy, phenyl, phenyl(C₁₋₄)alkyl, phenoxy, phenylthio, phenylS(O), phenylS(O)₂, phenyl(C₁₋₄)alkoxy, heteroaryl, heteroaryl(C₁₋₄)alkyl, heteroaryloxy or heteroaryl(C₁₋₄)alkoxy; wherein any of the immediately foregoing phenyl and heteroaryl moieties are optionally substituted with halo, hydroxy, nitro, S(C₁₋₄ alkyl), S(O)(C₁₋₄ alkyl), S(O)2(C₁₋₄ alkyl), S(O)₂NH₂, S(O)₂NH(C₁₋₄ alkyl), S(O)₂N(C₁₋₄ alkyl)₂, cyano, C₁₋₄ alkyl, C₁₋₄ alkoxy, C(O)NH₂, C(O)NH(C₁₋₄ alkyl), C(O)N(C₁₋₄ alkyl)₂, CO₂H, CO₂(C₁₋₄ alkyl), NHC(O)(C₁₋₄ alkyl), NHS(O)₂(C₁₋₄ alkyl), CF₃ or OCF₃;
unless otherwise stated heterocyclyl is optionally substituted by C₁₋₆ alkyl [optionally substituted by phenyl {which itself optionally substituted by halo, C₁₋₄ alkyl, C₁₋₄ alkoxy, cyano, nitro, CF₃, OCF₃, (C₁₋₄ alkyl)C(O)NH, S(O)₂NH₂, C₁₋₄ alkylthio, S(O)(C₁₋₄ alkyl) or S(O)₂(C₁₋₄ alkyl)} or heteroaryl {which itself optionally substituted by halo, C₁₋₄ alkyl, C₁₋₄ alkoxy, cyano, nitro, CF₃, (C₁₋₄ alkyl)C(O)NH, S(O)₂NH₂, C₁₋₄ alkylthio, S(O)(C₁₋₄ alkyl) or S(O)₂(C₁₋₄ alkyl)}], phenyl {optionally substituted by halo, C₁₄ alkyl, C₁₋₄ alkoxy, cyano, nitro, CF₃, OCF₃, (C₁₄ alkyl)C(O)NH, S(O)₂NH₂, C₁₋₄ alkylthio, S(O)(C₁₋₄ alkyl) or S(O)₂(C₁₋₄ alkyl)}, heteroaryl {optionally substituted by halo, C₁₋₄ alkyl, C₁₋₄ alkoxy, cyano, nitro, CF₃, (C₁₋₄ alkyl)C(O)NH, S(O)₂NH₂, C₁₋₄ alkylthio, S(O)(C₁₋₄ alkyl) or S(O)₂(C₁₋₄ alkyl)}, S(O)₂NR⁴⁰R⁴¹, C(O)R⁴², C(O)₂(C₁₋₆ alkyl) (such as tert-butoxycarbonyl), C(O)₂(phenyl(C₁₋₂ alkyl)) (such as benzyloxycarbonyl), C(O)NHR⁴³, S(O)₂R⁴⁴, NHS(O)₂NHR⁴⁵, NHC(O)R⁴⁶, NHC(O)NHR⁴⁷ or NHS(O)₂R⁴⁸, provided none of these last four substituents is linked to a ring nitrogen;
k, l and q are, independently, 0, 1 or 2;
R²⁰, R²², R²⁴, R²⁶, R²⁷, R²⁹, R³¹, R³³, R³⁷, R⁴⁰, and R⁵¹ are, independently, hydrogen or C₁₋₆ alkyl;
R²¹, R²³, R²⁵, R²⁸, R³⁰, R³², R³⁴, R³⁶, R³⁸, R³⁹, R⁴¹, R⁴², R⁴³, R⁴⁴, R⁴⁵, R⁴⁶, R⁴⁷, R⁴⁸, R⁴⁹, R⁵⁰ and R⁵² are, independently, C₁₋₆ alkyl (optionally substituted by halo, hydroxy, C₁₋₆ alkoxy, C₁₋₆ haloalkoxy, C₃₋₆ cycloalkyl, C₅₋₆ cycloalkenyl, S(C₁₋₄ alkyl), S(O)(C₁₋₄ alkyl), S(O)₂(C₁₋₄ alkyl), heteroaryl, phenyl, heteroaryloxy or phenyloxy), C₃₋₇ cycloalkyl, phenyl or heteroaryl; wherein any of the immediately foregoing phenyl and heteroaryl moieties are optionally substituted with halo, hydroxy, nitro, S(C₁₋₄ alkyl), S(O)(C₁₋₄ alkyl), S(O)₂(C₁₋₄ alkyl), S(O)₂NH₂, S(O)₂NH(C₁₋₄ alkyl), S(O)₂N(C₁₋₄ alkyl)₂, cyano, C₁₋₄ alkyl, C₁₋₄ alkoxy, C(O)NH₂, C(O)NH(C₁₋₄ alkyl), C(O)N(C₁₋₄ alkyl)₂, CO₂H, CO₂(C₁₋₄ alkyl), NHC(O)(C₁₋₄ alkyl), NHS(O)₂(C₁₋₄ alkyl), C(O)(C₁₋₄ alkyl), CF₃ or OCF₃;
R²¹, R²³, R²⁵, R²⁸, R³⁰, R³⁴, R³⁵, R³⁶, R⁴¹, R⁴², R⁴³, R⁴⁵, R⁴⁶, R⁴⁷ and R⁵² may additionally be hydrogen;
or a pharmaceutically acceptable salt thereof or a solvate thereof.

2. A compound as claimed in claim 1 wherein R¹ is NR¹³C(O)R¹⁴, wherein R¹³ and R¹⁴ are as defined in claim 1.

3. A compound as claimed in claim 1 or 2 wherein R¹ is optionally substituted aryl or optionally substituted heteroaryl, wherein the optional substituents are as recited in claim 1.

4. A compound as claimed in claim 1, 2 or 3 wherein R¹ is optionally substituted heterocyclyl.

5. A compound as claimed in any one of the preceding claims wherein R² is phenyl optionally substituted by halo or CF₃.

6. A compound as claimed in any one of the preceding claims wherein R³ is hydrogen.

7. A compound as claimed in any one of the preceding claims wherein R⁴ is heterocyclyl optionally substituted by oxo, halogen, cyano, hydroxy, C₁₋₆ alkyl (itself optionally substituted by halogen, hydroxy, cyano or C₁₋₄ alkoxy), C₂₋₄ alkenyl, CO₂(C₁₋₄ alkyl), S(O)₂(C₁₋₄ alkyl), CH(O), S(O)₂(C₁₋₄ haloalkyl), C(O)(C₁₋₄ alkyl), C(O)(C₃₋₆ cycloalkyl), N(C₁₋₄ alkyl)₂, C(O)NH₂, C(O)N(C₁₋₄ alkyl)₂ or NHC(O)(C₁₋₄ alkyl).

8. A compound as claimed in any one of the preceding claims wherein heterocyclyl is piperidinyl, homopiperazinyl, thiomorpholinyl, pyrrolidinyl, piperazinyl, 1,2,3,6-tetrahydropyridinyl, morpholinyl, 2,5-dihydropyrrolyl, azetidinyl, 1,4-oxepanyl, 3-azabicyclo[3.2.1]octan-3-yl, 8-azaspiro[4.5]decanyl or 3-azabicyclo[3.1.0]hex-3-yl.

9. A compound as claimed in any one of the preceding claims wherein A is absent.

10. A compound as claimed in any one of the preceding claims wherein n is 2.

11. A process for preparing a compound as claimed in claim 1, the process comprising:
i. when R¹ is an N-linked optionally substituted heterocycle, reacting a compound of formula (II): 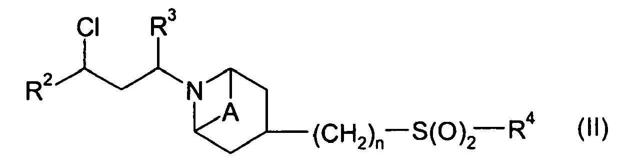 wherein R², R³, R⁴, n, A and X are as defined in claim 1, with a compound R¹H (wherein the H is on a heterocycle ring nitrogen atom) wherein R¹ is as defined in claim 1, in the presence of a suitable base and in a suitable solvent;
ii. when R³ is hydrogen, coupling a compound of formula (III): 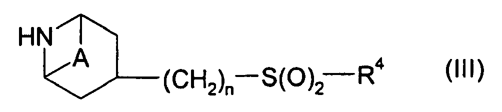 wherein R⁴, n, A and X are as defined in claim 1, with a compound of formula (IV): 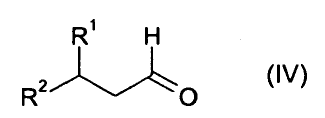 wherein R¹ and R² are as defined in claim 1, in the presence of NaBH(OAc)₃ (wherein Ac is C(O)CH₃) in a suitable solvent at room temperature; or,
iii. when R³ is hydrogen, coupling a compound of formula (III): 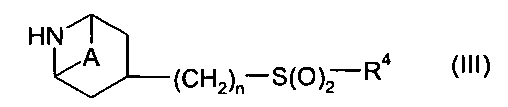 wherein R⁴, n, A and X are as defined in claim 1, with a compound of formula (V): 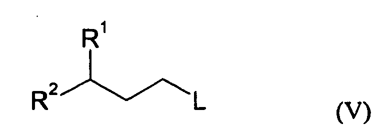 wherein R¹ and R² are as defined in claim 1 and L is an activated leaving group, in the presence of a base, in a suitable solvent at a temperature from 60°C up to the boiling point of the solvent.

12. A pharmaceutical composition which comprises a compound as claimed in claim 1, or a pharmaceutically acceptable salt thereof or solvate thereof, and a pharmaceutically acceptable adjuvant, diluent or carrier.

13. A compound as claimed in claim 1, or a pharmaceutically acceptable salt thereof or solvate thereof, for use as a medicament.

14. A compound as claimed in claim 1, or a pharmaceutically acceptable salt thereof or solvate thereof, in the manufacture of a medicament for use in therapy.

## Patentansprüche

1. Verbindung der Formel (I): 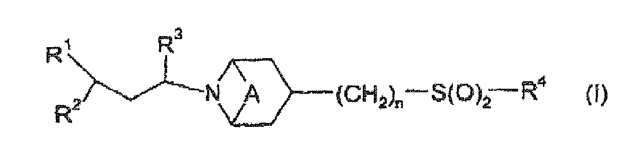 worin:
A fehlt oder für (CH₂)₂ steht;
R¹ für C(O)NR¹⁰R¹¹, C(O)₂R¹², NR¹³C(O)R¹⁴, NR¹⁵C(O)NR¹⁶R¹⁷, NR¹⁸C(O)₂R¹⁹, Heterocyclyl, Aryl oder Heteroaryl steht;
R¹⁰, R¹³, R¹⁵, R¹⁶ und R¹⁸ für Wasserstoff oder C₁₋₆-Alkyl stehen;
R¹¹, R¹², R¹⁴, R¹⁷ und R¹⁹ für C₁₋₈-Alkyl (gegebenenfalls substituiert durch Halogen, Hydroxy, C₁₋₆-Alkoxy, C₁₋₆-Halogenalkoxy, C₃₋₆-Cycloalkyl (gegebenenfalls substituiert durch Halogen), C₅₋₆-Cycloalkenyl, S(C₁₋₄-Alkyl), S(O)(C₁₋₄-Alkyl), S (O)₂(C₁₋₄-Alkyl), Heteroaryl, Aryl, Heteroaryloxy oder Aryloxy), Aryl, Heteroaryl, C₃₋₇-Cycloalkyl (gegebenenfalls substituiert durch Halogen oder C₁₋₄-Alkyl), an einen Phenylring anelliertes C₄₋₇-Cycloalkyl, C₅₋₇-Cycloalkenyl oder Heterocyclyl (selbst gegebenenfalls substituiert durch Oxo, C(O)(C₁₋₆-Alkyl), S(O)ₖ(C₁₋₆-Alkyl), Halogen oder C₁₋₄-Alkyl) stehen, oder R¹¹, R¹², -R¹⁴ und _{R}¹⁷ auch für Wasserstoff stehen können; oder R¹⁰ und R¹¹ und/oder R¹⁶ und R¹⁷ gemeinsam einen 4-, 5- oder 6-gliedrigen Ring bilden können, der gegebenenfalls ein Stickstoff-, Sauerstoff- oder Schwefelatom enthält und gegebenenfalls durch C₁₋₆-Alkyl, S (O)₁(C₁₋₆-Alkyl) oder C (O) (C₁₋₆-Alkyl) substituiert ist;
R² für Phenyl, Heteroaryl oder C₃₋₇-Cycloalkyl steht;
R³ für H oder C₁₋₄-Alkyl steht;
R⁴ für Heterocyclyl steht;
n für 1, 2 oder 3 steht;
Aryl-, Phenyl- und Heteroarylgruppierungen unabhängig voneinander gegebenenfalls ein- oder mehrfach durch Halogen, Cyano, Nitro, Hydroxy, OC(O)NR²⁰R²¹, NR²²R²³, NR²⁴C(O)R²⁵, NR²⁶C(O)NR²⁷R²⁸, S(O)₂NR²⁹R³⁰, NR³¹S(O)₂R³², C(O)NR³³R³⁴, CO₂R³⁶, NR³⁷CO₂R³⁸, S(O)_{q}R³⁹, OS(O)₂R⁴⁹, C₁₋₆-Alkyl (gegebenenfalls einfach substituiert durch S(O)₂R⁵⁰ oder C (O) NR⁵¹R⁵²), C₂₋₆-Alkenyl, C₂₋₆-Alkinyl, C₃₋₁₀-Cycloalkyl, C₁₋₆-Halogenalkyl, C₁₋₆-Alkoxy-C₁₋₆-alkyl, C₁₋₆-Alkoxy, C₁₋₆-Halogenalkoxy, Phenyl, Phenyl-C₁₋₄-alkyl, Phenoxy, Phenylthio, Phenyl-S(O), Phenyl-S(O)₂, Phenyl-C₁₋₄-alkoxy, Heteroaryl, Heteroaryl-C₁₋₄-alkyl, Heteroaryloxy oder Heteroaryl-C₁₋₄-alkoxy substituiert sind; wobei jede der unmittelbar vorstehenden Phenyl- und Heteroarylgruppierungen gegebenenfalls durch Halogen, Hydroxy, Nitro, S(C₁₋₄-Alkyl), S(O)(C₁₋₄-Alkyl), S(O)₂(C₁₋₄-Alkyl), S(O)₂NH₂, S(O)₂NH(C₁₋₄-Alkyl), S(O)₂N(C₁₋₄-Alkyl)₂, Cyano, C₁₋₄-Alkyl, C₁₋₄-Alkoxy, C(O)NH₂, C(O)NH(C₁₋₄-Alkyl), C(O)N(C₁₋₄-Alkyl)₂, CO₂H, CO₂(C₁₋₄-Alkyl), NHC(O)(C₁₋₄-Alkyl), NHS(O)₂(C₁₋₄-Alkyl), CF₃ oder OCF₃ substituiert ist; sofern nicht anders vermerkt, Heterocyclyl gegebenenfalls durch C₁₋₆-Alkyl [gegebenenfalls substituiert durch Phenyl {welches gegebenenfalls selbst durch Halogen, C₁₋₄-Alkyl, C₁₋₄-Alkoxy, Cyano, Nitro, CF₃, OCF₃, (C₁₋₄-Alkyl)C(O)NH, S(O)₂NH₂, C₁₋₄-Alkylthio, S(O)(C₁₋₄-Alkyl) oder S(O)₂(C₁₋₄-Alkyl) substituiert ist} oder Heteroaryl {welches gegebenenfalls selbst durch Halogen, C₁₋₄-Alkyl, C₁₋₄-Alkoxy, Cyano, Nitro, CF₃, (C₁₋₄-Alkyl)C(O)NH, S(O)₂NH₂, C₁₋₄-Alkylthio, S(O)(C₁₋₄-Alkyl) oder S(O)₂(C₁₋₄-Alkyl) substituiert ist}], Phenyl {gegebenenfalls substituiert durch Halogen, C₁₋₄-Alkyl, C₁₋₄-Alkoxy, Cyano, Nitro, CF₃, OCF₃, (C₁₋₄-Alkyl) C (O) NH, S(O)₂NH₂, C₁₋₄-Alkylthio, S(O)(C₁₋₄-Alkyl) oder S(O)₂(C₁₋₄-Alkyl)}, Heteroaryl {gegebenenfalls substituiert durch Halogen, C₁₋₄-Alkyl, C₁₋₄-Alkoxy, Cyano, Nitro, CF₃, (C₁₋₄-Alkyl)C(O)NH, S(O)₂NH₂, C₁₋₄-Alkylthio, S(O)(C₁₋₄-Alkyl) oder S(O)₂(C₁₋₄-Alkyl)}, S(O)₂NR⁴⁰R⁴¹, C(O)R⁴², C(O)₂(C₁₋₆-Alkyl) (wie tert.-Butoxyacarbonyl), C(O)₂(Phenyl(C₁₋₂-alkyl)) (wie Benzyloxycarbonyl), C(O)NHR⁴³, S(O)₂R⁴⁴, NHS(O)₂NHR⁴⁵, NHC(O)R⁴⁶, NHC(O)NHR⁴⁷ oder NHS(O)₂R⁴⁸ substituiert ist, mit der Maßgabe, daß keiner der letzten vier Substituenten an einen Ringstickstoff gebunden ist;
k, l und q unabhängig voneinander für 0, 1 oder 2 stehen;
R²⁰, R²², R²⁴, R²⁶, R²⁷, R²⁹, R³¹, R³³, R³⁷, R⁴⁰ und R⁵¹ unabhängig voneinander für Wasserstoff oder C₁₋₆-Alkyl stehen;
R²¹, R²³, R²⁵, R²⁸, R³⁰, R³², R³⁴, R³⁶, R³⁸, R³⁹, R⁴¹, R⁴², R⁴³, R⁴⁴, R⁴⁵, R⁴⁶, R⁴⁷, R⁴⁸, R⁴⁹, R⁵⁰ und R⁵² unabhängig voneinander für C₁₋₆-Alkyl (gegebenenfalls substituiert durch Halogen, Hydroxy, C₁₋₆-Alkoxy, C₁₋₆-Halogenalkoxy, C₃₋₆-Cycloalkyl, C₅₋₆-Cycloalkenyl, S(C₁₋₄-Alkyl), S(O)(C₁₋₄-Alkyl), S(O)₂(C₁₋₄Alkyl), Heteroaryl, Phenyl, Heteroaryl oder Phenyloxy), C₃₋₇-Cycloalkyl, Phenyl oder Heteroaryl stehen; wobei jede der unmittelbar vorstehenden Phenyl- und Heteroarylgruppierungen gegebenenfalls durch Halogen, Hydroxy, Nitro, S(C₁₋₄-Alkyl), S(O)(C₁₋₄-Alkyl), S(O)₂(C₁₋₄-Alkyl), S(O)₂NH₂, S(O)₂NH (C₁₋₄-Alkyl), S(O)₂N(C₁₋₄-Alkyl)₂, Cyano, C₁₋₄-Alkyl, C₁₋₄-Alkoxy, C(O)NH₂, C(O)NH(C₁₋₄-Alkyl), C(O)N(C₁₋₄-Alkyl) ₂, CO₂H, CO₂(C₁₋₄-Alkyl), NHC(O)(C₁₋₄-Alkyl), NHS(O)₂(C₁₋₄-Alkyl), C(O)(C₁₋₄-Alkyl), CF₃ oder OCF₃ substituiert ist;
R²¹, R²³, R²⁵ , R²⁸ , R³⁰ , R³⁴ , R³⁵, R³⁶, R⁴¹ , R⁴² , R⁴³ , R⁴⁵, R⁴⁶, R⁴⁷ und _{R}⁵² zusätzlich auch für Wasserstoff stehen können;
oder ein pharmazeutisch annehmbares Salz davon oder ein Solvat davon.

2. Verbindung nach Anspruch 1, in der R¹ für NR¹³C(O)R¹⁴ steht, wobei R¹³ und R¹⁴ die in Anspruch 1 angegebene Bedeutung besitzen.

3. Verbindung nach Anspruch 1 oder 2, in der R¹ für gegebenenfalls substituiertes Aryl oder gegebenenfalls substituiertes Heteroaryl steht, wobei die fakultativen Substituenten den Angaben in Anspruch 1 entsprechen.

4. Verbindung nach Anspruch 1, 2 oder 3, in der R¹ für gegebenenfalls substituiertes Heterocyclyl steht.

5. Verbindung nach einem der vorhergehenden Ansprüche, in der R² für gegebenenfalls durch Halogen oder CF₃ substituiertes Phenyl steht.

6. Verbindung nach einem der vorhergehenden Ansprüche, in der R³ für Wasserstoff steht.

7. Verbindung nach einem der vorhergehenden Ansprüche, in der R⁴ für gegebenenfalls durch Oxo, Halogen, Cyano, Hydroxy, C₁₋₆-Alkyl (selbst gegebenenfalls substituiert durch Halogen, Hydroxy, Cyano oder C₁₋₄-Alkoxy), C₂₋₄-Alkenyl, CO₂(C₁₋₄-Alkyl), S(O)₂(C₁₋₄-Alkyl), CH(O), S (O)₂(C₁₋₄-Halogenalkyl), C(O)(C₁₋₄-Alkyl), C(O)(C₃₋₆-Cycloalkyl), N(C₁₋₄-Alkyl)₂, C(O)NH₂, C(O)N(C₁₋₄-Alkyl)₂ oder NHC(O)(C₁₋₄-Alkyl) substituiertes Heterocyclyl steht.

8. Verbindung nach einem der vorhergehenden Ansprüche, in der Heterocyclyl für Piperidinyl, Homopiperazinyl, Thiomorpholinyl, Pyrrolidinyl, Piperazinyl, 1,2,3,6-Tetrahydropyridinyl, Morpholinyl, 2,5-Dihydropyrrolyl, Azetidinyl, 1,4-Oxepanyl, 3-Azabicyclo[3.2.1]octan-3-yl, 8-Azaspiro[4.5]decanyl oder 3-Azabicyclo[3.1.0]hex-3-yl steht.

9. Verbindung nach einem der vorhergehenden Ansprüche, in der A fehlt.

10. Verbindung nach einem der vorhergehenden Ansprüche, in der n für 2 steht.

11. Verfahren zur Herstellung einer Verbindung nach Anspruch 1, bei dem man:
i. wenn R¹ für einen N-verknüpften gegebenenfalls substituierten Heterocyclus steht, eine Verbindung der Formel (II): 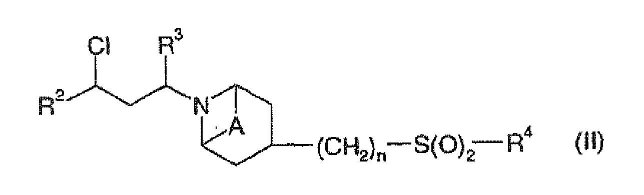 worin R², R³, R⁴, n, A und X die in Anspruch 1 angegebene Bedeutung besitzen, in Gegenwart einer geeigneten Base in einem geeigneten Lösungsmittel mit einer Verbindung R¹H (worin sich das H an einem Ringstickstoffatom eines Heterocyclus befindet), worin R¹ die in Anspruch 1 angegebene Bedeutung besitzt, umsetzt;
ii. wenn R³ für Wasserstoff steht, eine Verbindung der Formel (III): 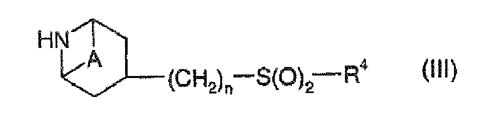 worin R⁴, n, A und X die in Anspruch 1 angegebene Bedeutung besitzen, in Gegenwart von NaBH(OAc)₃ (worin Ac für C(O)CH₃ steht) in einem geeigneten Lösungsmittel bei Raumtemperatur mit einer Verbindung der Formel (IV): 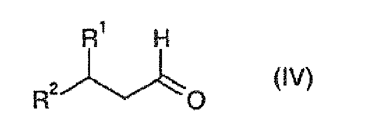 worin R¹ und R² die in Anspruch 1 angegebene Bedeutung besitzen, kuppelt;
iii. wenn R³ für Wasserstoff steht, eine Verbindung der Formel (III): 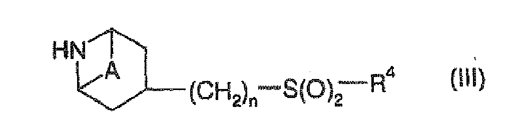 worin R⁴, n, A und X die in Anspruch 1 angegebene Bedeutung besitzen, in Gegenwart einer Base in einem geeigneten Lösungsmittel bei einer Temperatur von 60°C bis zum Siedepunkt des Lösungsmittels mit einer Verbindung der Formel (V): 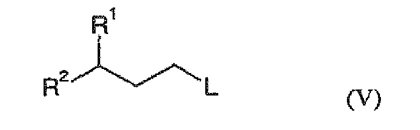 worin R¹ und R² die in Anspruch 1 angegebene Bedeutung besitzen und L für eine aktivierte Abgangsgruppe steht, kuppelt.

12. Pharmazeutische Zusammensetzung, die eine Verbindung nach Anspruch 1 oder ein pharmazeutisch annehmbares Salz davon oder Solvat davon und einen pharmazeutisch annehmbaren Hilfsstoff, ein pharmazeutisch annehmbares Verdünnungsmittel oder einen pharmazeutisch annehmbaren Träger enthält.

13. Verbindung nach Anspruch 1 oder ein pharmazeutisch annehmbares Salz davon oder Solvat davon zur Verwendung als Arzneimittel.

14. Verwendung einer Verbindung nach Anspruch 1 oder eines pharmazeutisch annehmbaren Salzes davon oder Solvats davon bei der Herstellung eines Arzneimittels zur Verwendung bei der Therapie.

## Revendications

1. Composé de formule (I) : 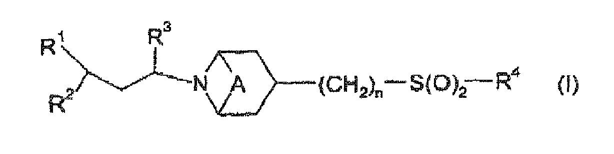 dans laquelle :
A est absent ou est (CH₂) ₂ ;
R¹ est C(O)NR¹⁰R¹¹, C(O)₂R¹², NR¹³C(O)R¹⁴, NR¹⁵C(O)NR¹⁶R¹⁷, NR¹⁸C(O)₂R¹⁹, hétérocyclyle, aryle ou hétéroaryle ;
R¹⁰, R¹³, R¹⁵, R¹⁶ et R¹⁸ sont hydrogène ou C₁₋₆-alkyle ;
R¹¹, R¹², R¹⁴, R¹⁷ et _{R}¹⁹ sont C₁₋₈-alkyle (éventuellement substitué par halogéno, hydroxy, C₁₋₆-alcoxy, C₁₋₆-halogénoalcoxy, C₃₋₆-cycloalkyle (éventuellement substitué par halogéno), C₅₋₆-cycloalcényle, S(C₁₋₄-alkyle), S(O)(C₁₋₄-alkyle), S(O)₂(C₁₋₄-alkyle), hétéroaryle, aryle, hétéroaryloxy ou aryloxy), aryle, hétéroaryle, C₃₋₇-cycloalkyle (éventuellement substitué par halogéno ou C₁₋₄-alkyle), C₄₋₇-cycloalkyle condensé sur un cycle phényle, C₅₋₇-cycloalcényle ou hétérocyclyle (lui-même éventuellement substitué par oxo, C(O)(C₁₋₆-alkyle), S(O)ₖ(C₁₋₆-alkyle), halogéno ou C₁₋₄-alkyle) ; ou R¹¹, R¹², R¹⁴ et R¹⁷ peuvent également être hydrogène ;
ou R¹⁰ et R¹¹ et/ou R¹⁶ et R¹⁷ peuvent se lier pour former un cycle à 4, 5 ou 6 chaînons qui peuvent éventuellement inclure un atome d'azote, d'oxygène ou de soufre, ledit cycle étant éventuellement substitué par C₁₋₆-alkyle, S(O)₁(C₁₋₆-alkyle) ou C(O)(C₁₋₆-alkyle);
R² est phényle, hétéroaryle ou C₃₋₇-cycloalkyle ;
R³ est H ou C₁₋₄-alkyle ;
R⁴ est hétérocyclyle ;
n est 1, 2 ou 3 ;
les motifs aryle, phényle et hétéroaryle sont, indépendamment, éventuellement substitués par un ou plusieurs parmi halogéno, cyano, nitro, hydroxy, OC(O)NR²⁰R²¹, NR²²R²³, NR²⁴C(O)R²⁵, NR²⁶C (O) NR²⁷R²⁸, S(O)₂NR²⁹R³⁰, NR³¹S(O)₂R³², C(O)NR³³R³⁴, CO₂R³⁶, NR³⁷CO₂R³⁸, S(O)_{q}R³⁹, OS(O)₂R⁴⁹, C₁₋₆-alkyle (éventuellement monosubstitué par S(O)₂R⁵⁰ ou C(O)NR⁵¹R⁵²), C₂₋₆-alcényle, C₂₋₆-alcynyle, C₃₋₁₀-cycloalkyle, C₁₋₆-halogénoalkyle, C₁₋₆-alcoxy (C₁₋₆)-alkyle, C₁₋₆-alcoxy, C₁₋₆-halogénoalcoxy, phényle, phényl(C₁₋₄) alkyle, phénoxy, phénylthio, phényl-S(O), phényl-S(O)₂, phényl(C₁₋₄)alcoxy, hétéroaryle, hétéroaryl(C₁₋₄)alkyle, hétéroaryloxy ou hétéroaryl(C₁₋₄)alcoxy ; où l'un quelconque des motifs phényle et hétéroaryle venant d'être cités sont éventuellement substitués par halogéno, hydroxy, nitro, S(C₁₋₄-alkyle), S(O)(C₁₋₄-alkyle), S(O)₂(C₁₋₄-alkyle), S(O)₂NH₂, S(O)₂NH(C₁₋₄-alkyle), S(O)₂N(C₁₋₄-alkyle)₂, cyano, C₁₋₄-alkyle, C₁₋₄-alcoxy, C(O)NH₂, C(O)NH(C₁₋₄-alkyle), C(O)N(C₁₋₄-alkyle)₂, CO₂H, CO₂(C₁₋₄-alkyle), NHC(O)(C₁₋₄-alkyle), NHS(O)₂(C₁₋₄-alkyle), CF₃ ou OCF₃ ;
sauf indication contraire, hétérocyclyle est éventuellement substitué par C₁₋₆-alkyle [éventuellement substitué par phényle {qui est lui-même éventuellement substitué par halogéno, C₁₋₄-alkyle, C₁₋₄-alcoxy, cyano, nitro, CF₃, OCF₃, (C₁₋₄-alkyle)C(O)NH, S(O)₂NH₂, C₁₋₄-alkylthio, S (O) (C₁₋₄-alkyle) ou S (O) ₂ (C₁₋₄-alkyle)} ou hétéroaryle {qui est lui-même éventuellement substitué par halogéno, C₁₋₄-alkyle, C₁₋₄-alcoxy, cyano, nitro, CF₃, (C₁₋₄-alkyle)C(O)NH, S(O)₂NH₂, C₁₋₄-alkylthio, S (O) (C₁₋₄-alkyle) ou S (O) ₂ (C₁₋₄-alkyle)}], phényle {éventuellement substitué par halogéno, C₁₋₄-alkyle, C₁₋₄-alcoxy, cyano, nitro, CF₃, OCF₃, (C₁₋₄-alkyle) C(O)NH, S(O)₂NH₂, C₁₋₄-alkylthio, S(O)(C₁₋₄-alkyle) ou S(O)₂(C₁₋₄-alkyle)}, hétéroaryle {éventuellement substitué par halogéno, C₁₋₄-alkyle, C₁₋₄-alcoxy, cyano, nitro, CF₃, (C₁₋₄-alkyle)C(O)NH, S(O)₂NH₂, C₁₋₄-alkylthio, S(O)(C₁₋₄-alkyle) ou S(O)₂(C₁₋₄-alkyle)}, S(O)₂NR⁴⁰R⁴¹, C(O)R⁴², C(O)₂(C₁₋₆-alkyle) (tel que tert-butoxycarbonyle), C(O)₂(phényl(C₁₋₂-alkyle)) (tel que benzyloxy-carbonyle), C(O)NHR⁴³, S(O)₂R⁴⁴, NHS(O)₂NHR⁴⁵, NHC(O)R⁴⁶, NHC(O)NHR⁴⁷ ou NHS(O)₂R⁴⁸, à condition qu'aucun de ces quatre derniers substituants ne soit lié à un atome d'azote du cycle ;
k, l et q sont, indépendamment, 0, 1 ou 2 ;
R²⁰ , R²² , R²⁴ , R²⁶ , R²⁷ , R²⁹ , R³¹ , R³³ , R³⁷ , R⁴⁰ et R⁵¹ sont, indépendamment, hydrogène ou C₁₋₆-alkyle ;
R²¹ , R²³ , R²⁵ , R²⁸ , R³⁰ , R³² , R³⁴ , R³⁶ , R³⁸ , R³⁹ , R⁴¹ , R⁴², R⁴³, R⁴⁴, R⁴⁵, R⁴⁶, R⁴⁷, R⁴⁸, R⁴⁹, R⁵⁰ et R⁵² sont, indépendamment, C₁₋₆-alkyle (éventuellement substitué par halogéno, hydroxy, C₁₋₆-alcoxy, C₁₋₆-halogénoalcoxy, C₃₋₆-cycloalkyle, C₅₋₆-cycloalcényle, S(C₁₋₄-alkyle), S(O)(C₁₋₄-alkyle), S(O)₂(C₁₋₄alkyle), hétéroaryle, phényle, hétéroaryloxy ou phényloxy), C₃₋₇-cycloalkyle, phényle ou hétéroaryle ; où l'un quelconque des motifs phényle et hétéroaryle venant d'être cités sont éventuellement substitués par halogéno, hydroxy, nitro, S(C₁₋₄-alkyle), S(O)(C₁₋₄-alkyle), S(O)₂(C₁₋₄-alkyle), S(O)₂NH₂, S(O)₂NH(C₁₋₄-alkyle), S(O)₂N(C₁₋₄-alkyle)₂, cyano, C₁₋₄-alkyle, C₁₋₄-alcoxy, C(O)NH₂, C(O)NH(C₁₋₄-alkyle), C(O)N(C₁₋₄-alkyle)₂, CO₂H, CO₂(C₁₋₄-alkyle), NHC(O)(C₁₋₄-alkyle), NHS(O)₂(C₁₋₄-alkyle), C(O)C₁₋₄-alkyle), CF₃ ou OCF₃;
R²¹, R²³, R²⁵, R²⁸, R³⁰, R³⁴, R³⁵, R³⁶, R⁴¹, R⁴², R⁴³, R⁴⁵, R⁴⁶, R⁴⁷ et R⁵² peuvent en outre être hydrogène ;
ou sel pharmaceutiquement acceptable de celui-ci ou solvate de celui-ci.

2. Composé selon la revendication 1, dans lequel R¹ est NR¹³C(O)R¹⁴, où R¹³ et R¹⁴ sont tels que définis dans la revendication 1.

3. Composé selon la revendication 1 ou 2, dans lequel R¹ est aryle éventuellement substitué ou hétéroaryle éventuellement substitué, où les substituants éventuels sont tels qu'indiqués dans la revendication 1.

4. Composé selon la revendication 1, 2 ou 3, dans lequel R¹ est hétérocyclyle éventuellement substitué.

5. Composé selon l'une quelconque des revendications précédentes, dans lequel R² est phényle éventuellement substitué par halogéno ou CF₃.

6. Composé selon l'une quelconque des revendications précédentes, dans lequel R³ est hydrogène.

7. Composé selon l'une quelconque des revendications précédentes, dans lequel R⁴ est hétérocyclyle éventuellement substitué par oxo, halogène, cyano, hydroxy, C₁₋₆-alkyle (lui-même éventuellement substitué par halogène, hydroxy, cyano ou C₁₋₄-alcoxy), C₂₋₄-alcényle, CO₂(C₁₋₄-alkyle), S(O)₂(C₁₋₄-alkyle), CH(O), S(O)₂(C₁₋₄-halogénoalkyle), C(O)(C₁₋₄-alkyle), C(O)(C₃₋₆-cycloalkyle), N(C₁₋₄-alkyle)₂, C(O)NH₂, C(O)N(C₁₋₄-alkyle)₂ ou NHC(O)(C₁₋₄-alkyle).

8. Composé selon l'une quelconque des revendications précédentes, dans lequel hétérocyclyle est pipéridinyle, homopipérazinyle, thiomorpholinyle, pyrrolidinyle, pipérazinyle, 1,2,3,6-tétrahydropyridinyle, morpholinyle, 2,5-dihydropyrrolyle, azétidinyle, 1,4-oxépanyle, 3-azabicyclo[3,2,1]octan-3-yle, 8-azaspiro[4,5]décanyle ou 3-azabicyclo[3,1,0]hex-3-yle.

9. Composé selon l'une quelconque des revendications précédentes, dans lequel A est absent.

10. Composé selon l'une quelconque des revendications précédentes, dans lequel n est 2.

11. Procédé de préparation d'un composé selon la revendication 1, le procédé comprenant :
i. lorsque R¹ est un hétérocycle éventuellement substitué lié par N, la réaction d'un composé de formule (II) : 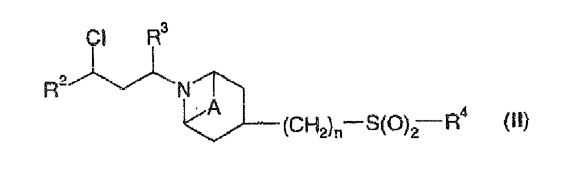 dans laquelle R², R³, R⁴, n, A et X sont tels que définis dans la revendication 1, avec un composé R¹H (où le H est sur un atome d'azote du cycle hétérocyclique), où R¹ est tel que défini dans la revendication 1, en présence d'une base appropriée et dans un solvant approprié ;
ii. lorsque R³ est hydrogène, le couplage d'un composé de formule (III) : 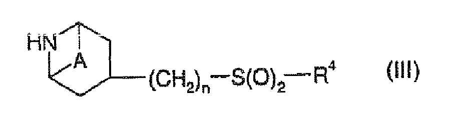 dans laquelle R⁴, n, A et X sont tels que définis dans la revendication 1, avec un composé de formule (IV) : 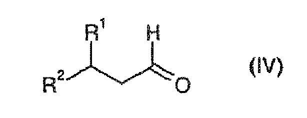 dans laquelle R¹ et R² sont tels que définis dans la revendication 1, en présence de NaBH(OAc)₃ (où Ac est C(O)CH₃) dans un solvant approprié à la température ambiante ; ou
iii. lorsque R³ est hydrogène, le couplage d'un composé de formule (III) : 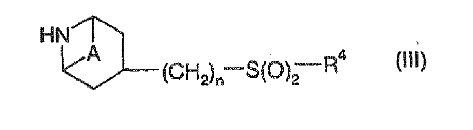 dans laquelle R⁴, n, A et X sont tels que définis dans la revendication 1, avec un composé de formule (V) : 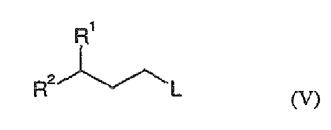 dans laquelle R¹ et R² sont tels que définis dans la revendication 1, et L est un groupement partant activé, en présence d'une base, dans un solvant approprié à une température de 60°C jusqu'au point d'ébullition du solvant.

12. Composition pharmaceutique comprenant un composé selon la revendication 1 ou un sel pharmaceutiquement acceptable de celui-ci, ou un solvate de celui-ci, et un adjuvant, un diluant ou un support pharmaceutiquement acceptable.

13. Composé selon la revendication 1 ou sel pharmaceutiquement acceptable de celui-ci ou solvate de celui-ci, destiné à être utilisé comme médicament.

14. Composé selon la revendication 1 ou sel pharmaceutiquement acceptable de celui-ci, ou solvate de celui-ci, dans la fabrication d'un médicament destiné à être utilisé en thérapie.
